# EUROPEAN PATENT SPECIFICATION

(11) **EP 4 389 201 B1**
(45) Date of publication and mention of the grant of the patent: **23.09.2026**
(21) Application number: 23217438.3
(22) Date of filing: 18.12.2023
(51) Int. Cl.: A61N 1/39

(54) **DEFIBRILLATION DEVICE, MEDICAL DEVICE AND RECORDING INSTRUMENT**
DEFIBRILLATIONSVORRICHTUNG, MEDIZINISCHE VORRICHTUNG UND AUFNAHMEINSTRUMENT
DISPOSITIF DE DÉFIBRILLATION, DISPOSITIF MÉDICAL ET INSTRUMENT D'ENREGISTREMENT

(30) Priority: 23.12.2022 CN 202211667385
(43) Date of publication of application: 26.06.2024
(73) Proprietor: Shenzhen Mindray Bio-Medical Electronics Co., Ltd., Shenzhen, Guangdong 518057 (CN)
(72) Inventor: LU, Yi, Shenzhen, 518057 (CN); YANG, Rui, Shenzhen, 518057 (CN); WAN, Wenjie, Shenzhen, 518057 (CN); LIU, Zongming, Shenzhen, 518057 (CN)
(74) Representative: KIPA AB

(56) References cited:
- WO-A1-2008/020369
- DE-A1- 102004 063 564
- US-A1- 2017 203 112
- US-A1- 2021 290 968
- US-A1- 2022 219 000

## Description

### TECHNICAL FIELD

The disclosure relates to the technical field of medical instrument, and more particularly to a defibrillation device, a medical device and a recording instrument.

### BACKGROUND

Defibrillator is an emergency device which is widely used in various public places and treatment rooms, mainly for timely defibrillation of patients with cardiac arrest caused by ventricular fibrillation.

In related technologies, defibrillators typically have a display component on its front surface for user to operate the defibrillator or view relevant information. However, the display component usually occupies a small proportion at the front surface of the defibrillator, resulting in a smaller area for user to operate or less information content displayed for user, which is not convenient for user to perform defibrillation on patient.

DE102004063564A1 discloses a device for the electromedical treatment of patients including three electromedical modules, which can be combined to a three-module device consisting of the three modules or to a two-module device.

### SUMMARY

An embodiment of this disclosure provides a defibrillation device, a medical device, and a recording instrument, which are capable of increasing a screen-to-body ratio thereof.

The invention is as defined in claims 1-15.

In embodiments of this disclosure, it can be understood that the first part is at the front surface of the host housing, while the second part is at other surfaces of the host housing, such as the bottom, top, left, right, or rear surfaces. At this time, the recording instrument outputs printing paper from the front surface of the host housing for the convenience of user. On this basis, compared to setting the entire recording instrument and display component side by side at the front surface of the host housing, embodiments of this disclosure hide at least a portion of the recording instrument at a rear side of the display component, and place into and take out from other surfaces of the host housing printing paper, such that a larger area at the front surface of the host housing can be provided for arranging the display component in embodiments of this disclosure, thereby ultimately improving the screen-to-body ratio of the display component. From this, it can be seen that the defibrillation device in embodiments of this disclosure has the advantage of a large screen-to-body ratio at the front surface, while making it easy for operator to obtain the printing paper which is printed.

### BRIEF DESCRIPTION OF THE DRAWINGS

The following is a detailed description of the specific implementation of this disclosure, combined with the accompanying drawings, which makes the technical solution and beneficial effects of this disclosure obvious.
FIG. 1 is a front view of a defibrillation device provided in an embodiment of this disclosure.
FIG. 2 is a cross-sectional view of the defibrillation device shown in FIG. 1.
FIG. 3 is a partially enlarged view of X in FIG. 2.
FIG. 4 is a structural diagram of an opening apparatus for a door body of the defibrillation device shown in FIG. 1.
FIG. 5 is a cross-sectional view of the defibrillation device shown in FIG. 4.
FIG. 6 is a partially enlarged view of Y in FIG. 5.
FIG. 7 is a structural diagram of a fitting structure between a recording instrument and a second part of the defibrillation device shown in FIG. 1.
FIG. 8 is a partially enlarged view of Z in FIG. 7.
FIG. 9 is an exploded view of a recording instrument and a door body of the defibrillation device shown in FIG. 1.
FIG. 10 is a cross-sectional view of a recording instrument and a door body shown in FIG. 4.
FIG. 11 is a first assembly diagram of a recording instrument and a door body of the defibrillation device shown in FIG. 1.
FIG. 12 is a structural diagram of a handle shown in FIG. 11.
FIG. 13 is a partial cross-sectional view of a handle and a door body shown in FIG. 10.
FIG. 14 is a second assembly diagram of a recording instrument and a door body of the defibrillation device shown in FIG. 1.
FIG. 15 is a 3D diagram of the defibrillation device shown in FIG. 1.
FIG. 16 is a block diagram of the defibrillation device shown in FIG. 15.
FIG. 17 is another front view of the defibrillation device shown in FIG. 1.

Reference numbers shown in the drawings have following meanings.
100. Host housing; 11. First part; 11a. First outer wall; 111. Fourth convex structure; 112. Sound output opening; 113. First opening; 114. First area; 12. Second part; 12a. Second outer wall; 121. Inward convex part; 1211. First inside surface; 1212. Second inside surface; 122. First convex structure; 1221. First convex rib; 1222. Second convex rib; 123. First fastening structure; 13. Third part; 14. Fourth part; 141. Holding structure; 15. Fifth part;
200. Display component; 21. Display structure layer; 22. Touch control layer;
300. Recording instrument; 31. Housing of recording instrument; 311. Second concave structure; 3111. First groove; 3112. Second groove; 312. First through-hole; 313. First position-limiting rib; 314. Second position-limiting rib; 315. First snap-fit; 32. Printing component; 33. First fastener; 34. First guide component; 341. Third groove; 35. Second guide component; 351. First insertion part; 352. Second insertion part; 353. Connection part; 3531. Third concave structure; 354. First paper output surface; 36. Paper roller;
400. Door body; 400a. Door body of recording instrument; 41. Handle; 411. Second snap-fit; 412. Abutting part; 413. Rotating shaft; 42. Second through-hole; 43. Second fastener; 44. Clamping part; 45. Second paper output surface; 451. Paper tearing structure; 452. Paper output opening; 46. Fixing structure for printing paper; 461. Circular lug boss;
500. Defibrillation module;
600. Parameter interface;
700. Treatment interface;
H1. First direction; H2. Second direction; H3. Third direction; H4. Fourth direction.

### DESCRIPTION OF THE PREFERRED EMBODIMENTS

The followings provide a clear and complete description of technical solutions in the embodiments of this disclosure combined with the accompanying drawings. Obviously, the described embodiments are only some of the embodiments of this disclosure, not all of them. Based on the embodiments in this disclosure, all other embodiments obtained by those skilled in the art without creative labor fall within the protection scope of this disclosure.

The embodiment of this disclosure provides a defibrillation device for timely defibrillation of patient with arrhythmia, such as atrial flutter, atrial fibrillation, supraventricular or ventricular tachycardia. Defibrillation device can be used in public places or in medical rooms, and the embodiments of this disclosure make no limitation for this.

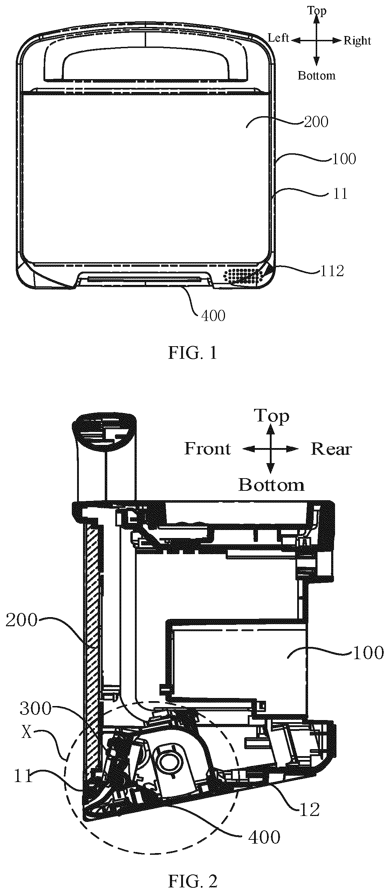

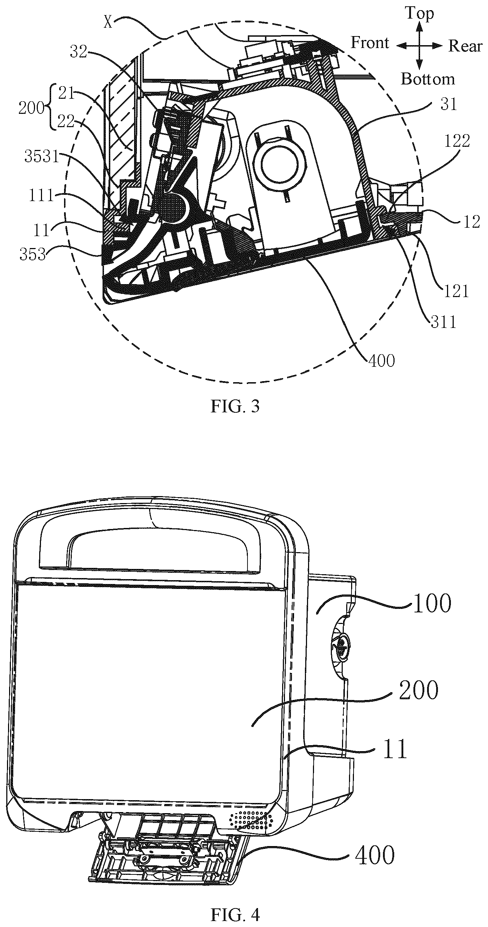

Please refer to FIGS. 1 to 3. FIG. 1 is a front view of a defibrillation device provided in an embodiment of this disclosure. FIG. 2 is a cross-sectional view of the defibrillation device shown in FIG. 1. FIG. 3 is a partially enlarged view of X in FIG. 2. The defibrillation device includes a host housing 100, a display component 200, a recording instrument 300, and a door body 400.

The host housing 100 serves as an outer housing for the defibrillation device. The host housing 100 includes a first part 11 at a first side and a second part 12 at a second side. That is to say, assuming that the first part 11 is located at a front surface of the host housing 100, or in other words, the first part 11 serves as a front wall of the host housing 100, then the second part 12 can be located at a bottom surface, a left surface, a right surface, a rear surface, or a top surface of the host housing 100, or in other words, the second part 12 servers as a bottom wall, a left wall, a right wall, a rear wall, or a top wall of the host housing 100. The embodiments of this disclosure make no limitation for this. Of course, depending on the shape and placement state of the host housing 100, the first part 11 may also be located at a tilted-up side or a tilted-down side of the host housing 100. Embodiments of this disclosure do not limit this. In the following description, embodiments of this disclosure is explained by taking the first part 11 located at the front surface of the host housing 100, or in other words, the first side being the front side of the host housing 100 as an example.

The display component 200 is assembled at the first part 11. That is to say, it can be understood that the display component 200 is assembled at the front surface or the front wall of the host housing 100.

The recording instrument 300 includes a housing 31 and a printing component 32. The housing 31 of the recording instrument is configured to accommodate printing paper. The housing 31 of the recording instrument is at least partially assembled at the host housing 100. The recording instrument 300 is configured such that the printing paper inside the housing 31 of the recording instrument is capable of being outputted to the first side of the host housing 100, or in other words, such that the printing paper inside the housing 31 of the recording instrument is capable of being outputted from the front surface of the host housing 100. The printing component 32 is assembled at the housing 31 of the recording instrument and is located at the host housing 100 for printing the printing paper, which is accommodated inside the housing 31 of the recording instrument. Therefore, the recording instrument 300 is capable of printing the printing paper and then outputting the printed paper from the front surface of the host housing 100, making it convenient for a user to obtain the printed paper outputted by the recording instrument 300.

The recording instrument 300 is further configured such that an orthographic projection of the recording instrument 300 on the first side at least partially overlaps with an orthographic projection of the display component 200 on the first side. Or in other words, an orthographic projection of the recording instrument 300 on a surface, where the display component 200 is located, at least partially overlaps with the display component 200. Therefore, it can also be understood that the recording instrument 300 is at least partially located behind the display component 200. So that, compared to setting the recording instrument 300 and the display component 200 side by side at the front surface of the host housing 100, as the recording instrument 300 is at least partially hidden behind the display component 200, the display component 200, which is located at the front surface of the host housing 100 in embodiments of this disclosure, can be made larger to increase the screen-to-body ratio of the front surface of the host housing 100.

The door body 400 is at least partially located at the second side of the host housing 100. The door body 400 is movably connected with the housing 31 of the recording instrument and/or the host housing 100, so as to enable the door body 400 to open or close an opening at the housing 31 of the recording instrument, thereby enabling the printing paper to be placed into or taken out from the housing 31 of the recording instrument from a side of the second part 12 of the host housing 100. That is to say, the door body 400 of the defibrillation device for replacing the printing paper is at least partially located in an area outside the front surface of the host housing 100, allowing the recording instrument 300 to replace the printing paper from the area outside the front surface of the host housing 100. Therefore, compared to reserving a larger area in the front surface of the host housing 100 for paper replacement, the display component 200, which is located at the front surface of the host housing 100 in the embodiment of this disclosure, can be made larger to increase the screen-to-body ratio at the front surface of the host housing 100.

Thus it can be seen, on the basis of outputting the printing paper from the front surface of the host housing 100 for the use to obtain the printing paper, the embodiment of this disclosure reserves more space at the front surface of the defibrillation device to assemble a larger display component 200 by hiding at least a portion of the recording instrument 300 behind the display component 200 and setting the recording instrument 300 to replace the printing paper from surfaces other than the front surface of the host housing 100, thereby increasing the screen-to-body ratio at the front surface of the defibrillation device. Finally, due to the assembly of the larger display component 200 at the front surface of the defibrillation device, the display component 200 displays more content, allowing user to obtain more interrelated information at once, thereby improving the defibrillation efficiency and convenience when using the defibrillation device.

As an example, that the orthogonal projection of the recording instrument 300 on the first side at least partially overlaps with the orthogonal projection of the display component 200 on the first side, includes, an orthogonal projection of the housing 31 of the recording instrument on the first side at least partially overlaps with the orthogonal projection of the display component 200 on the first side. In other words, the orthogonal projection of the housing 31 of the recording instrument on a plane, where the display component 200 is located, at least partially overlaps with the display component 200.

That is to say, in a vertical direction, the housing 31 of the recording instrument can be completely located at a rear side of the display component 200, or optionally a portion of the housing 31 of the recording instrument, such as four fifths, a half, or one third of the housing 31 of the recording instrument, is located at the rear side of the display component 200. This is not limited by the embodiments of this disclosure.

Optionally, that the orthogonal projection of the recording instrument 300 on the first side at least partially overlaps with the orthogonal projection of the display component 200 on the first side, includes, an orthogonal projection of the printing component 32 on the first side at least partially overlaps with the orthogonal projection of the display component 200 on the first side. In other words, the orthogonal projection of the printing component 32 on a plane, where the display component 200 is located, at least partially overlaps with the display component 200.

That is to say, in a vertical direction, the printing component 32 can be completely located at a rear side of the display component 200, or optionally a portion of the printing component 32, such as four fifths, a half, or one third of the printing component 32, is located at the rear side of the display component 200. This is not limited by the embodiments of this disclosure.

It can also be understood that, it is possible that just the orthogonal projection of the housing 31 of the recording instrument on the first side at least partially overlaps with the orthogonal projection of the display component 200 on the first side; or just the orthogonal projection of the printing component 32 on the first side at least partially overlaps with the orthogonal projection of the display component 200 on the first side; or both the orthogonal projection of the housing 31 of the recording instrument and the orthogonal projection of the printing component 32 on the first side at least partially overlap with the orthogonal projection of the display component 200 on the first side. This is not limited by the embodiments of this disclosure.

It can also be understood that, taking the display component 200 as a touch control panel, the touch control panel can include a display structure layer 21 and a touch control layer 22 located outside the display structure layer 21. The display structure layer 21 is used for imaging and displaying information. For example, the display structure layer 21 can be a display structure layer of liquid crystal, including a backlight module, an optical film layer, an array substrate, and a color filter film layer, or the display structure layer 21 can be an OLEDM (Organic Light Emitting Diode) display layer, etc. The embodiments of this disclosure make no limitation for this. The touch control layer 22 is configured to receive touch operations. For example, the touch control layer 22 can be a transparent cover plate provided with a capacitive touch control structure, a resistive touch control structure, or an infrared touch control structure.

It can be understood that an orthographic projection of the display structure layer 21 on the first side is usually smaller than an orthographic projection of the touch control layer 22 on the first side. In additional, the orthographic projection of display structure layer 21 on the first side is located within the orthographic projection of the touch control layer 22 on the first side. In other words, the touch control layer 22 typically has a larger area than the display structure layer 21 and covers the entire display structure layer 21.

At this point, it is possible that the orthographic projection of the recording instrument 300 on the first side just at least partially overlaps with the orthographic projection of the touch control layer 22 on the first side, or the orthographic projection of the recording instrument 300 on the first side further at least partially overlaps with the orthographic projection of the display structure layer 21 on the first side, which is not limited by embodiments of this disclosure.

In some embodiments, in order to make the overall structure of the defibrillation device more compact, the recording instrument 300 can be partially arranged side by side with the display structure layer 21 at an inside surface of the touch control layer 22, and partially located at a side of the display structure layer 21 facing away from the touch control layer 22. It can also be understood that the recording instrument 300 is partially located at a lower side of the display structure layer 21 and partially at a rear side of the display structure layer 21, which can fully utilize an area behind the touch control layer 22 where no display structure layer 21 is arranged, thus ultimately making the defibrillation device as a whole smaller.

As an example, the printing component 32 of the recording instrument 300 can be partially arranged side by side with the display structure layer 21 at the inside surface of the touch control layer 22, and partially located at a side of the display structure layer 21 facing away from the touch control layer 22. Furthermore, compared to that the entire printing component 32 is located at one side of the display structure layer 21 facing away from the touch control layer 22, the printing component 32 can be closer to the outer surface of the first part 11. Such that a paper output path for the printing paper to be outputted from the printing component 32 to the outside of the host housing 100 is shorter, thereby reducing the blockage probability of the printing paper inside the host housing 100.

Alternatively, the printing component 32 can also be arranged as a whole at one side of the display structure layer 21 facing away from the touch control layer 22, and embodiments of this disclosure do not limit this.

The above are some rough examples of the overall position of the recording instrument 300 in the embodiment of this disclosure. Below, a distance explanation is given for the technical solution of the embodiment of this disclosure in combination with the host housing 100 and some connecting and fitting structures of the recording instrument 300.

In some embodiments, in order to improve the protection of internal components of the host housing 100, a part which connects with the host housing 100 and the housing 31 of the recording instrument may be provided with a corresponding seal structure.

For example, the housing 31 of the recording instrument can only penetrate through the second part 12 to be assembled and fixed at the host housing 100, such that the seal structure can be arranged between the second part 12 and the housing 31 of the recording instrument. Optionally, the housing 31 of the recording instrument can also be connected and fitted with the second part 12 and the first part 11, respectively. Therefore, a connection part between the second part 12 and the housing 31 of the recording instrument can be provided with a seal structure, and a connection part between the first part 11 and the housing 31 of the recording instrument can also be provided with the seal structure. Embodiments of this disclosure do not limit this.

As an example, the second part 12 includes an inward convex part 121 extending from its outer surface toward an interior of the host housing 100. At least a portion of the inward convex part 121 is provided with a first convex structure 122 or a first concave structure (not shown in the figure), and the housing 31 of the recording instrument is provided with a second concave structure 311 or a second convex structure that fits with the first convex structure 122 or the first concave structure to seal between the housing 31 of the recording instrument and the second part 12.

On the one hand, the fitting between the first convex structure 122 or the first concave structure and the second concave structure 311 or the second convex structure can refer to a tight snap-in connection to achieve a seal, or a throttling gap formed therebetween so as to form a labyrinth seal structure. The embodiments of this disclosure are not limited to this.

On the other hand, it can be that the inward convex part 121 is provided with a first convex structure 122, the housing 31 of the recording instrument is provided with a second concave structure 311, and the first convex structure 122 is inserted into the second concave structure 311. It can also be that the inward convex part 121 is provided with a first concave structure, the housing 31 of the recording instrument is provided with a second convex structure, and the second convex structure is inserted (such as snap-in connection or clearance fitting) into the first concave structure. It can also be that the inward convex part 121 is provided with a first convex structure 122, and the housing 31 of the recording instrument is provided with a second convex structure. Along a direction in which the outer surface of the second part 12 faces inwardly, the first convex structure 122 and the second convex structure are misaligned with each other, forming an interface between adjacent surfaces of the inward convex part 121 and the housing 31 of the recording instrument, which interface extends inwardly from the outer surface of the second part 12 in a zigzagged way, thus ultimately achieving a seal and waterproof effect.

Of course, in some other embodiments, the inward convex part 121 can also be provided with both a first convex structure 122 and a first concave structure. Along a direction in which the outer surface of the second part 12 faces inwardly, the first convex structure 122 and the first concave structure are misaligned with each other. For example, the first convex structure 122 is located below the first concave structure, or the first concave structure is located below the first convex structure 122. Correspondingly, the housing 31 of the recording instrument is also provided with a second convex structure and a second concave structure 311. Furthermore, the first convex structure 122 is snap-fitted to the second concave structure 311 to form a first seal in a vertical direction, and the second convex structure is snap-fitted the first concave structure to form a second seal in the vertical direction. Alternatively, the first convex structure 122 is inserted into the second concave structure 311, and the second convex structure is inserted into the first concave structure, so that the first convex structure 122, the first concave structure, the second convex structure, and the second concave structure 311 cooperate to form a labyrinth seal.

It can be understood that any concave-convex fitting structure, which seals between the housing 31 of the recording instrument and the second part 12, is within the protection scope of embodiments of this disclosure, embodiments of this disclosure do not limit this.

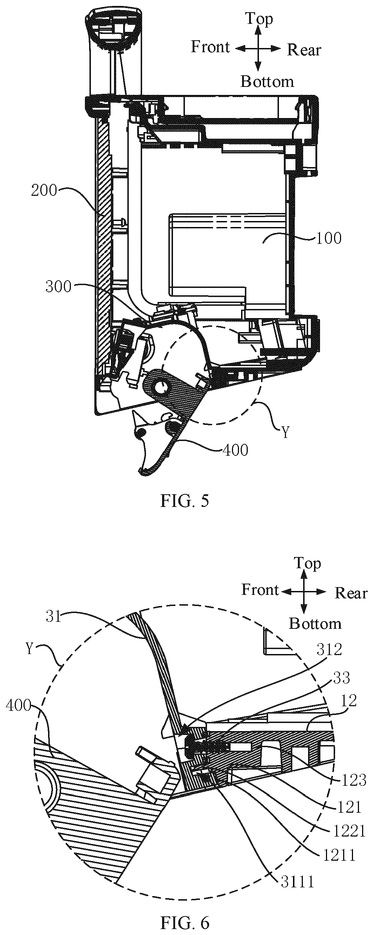

Please continue to refer to FIGS. 4 to 6. FIG. 4 is a structural diagram of an opening apparatus for a door body of the defibrillation device shown in FIG. 1. FIG. 5 is a cross-sectional view of the defibrillation device shown in FIG. 4. FIG. 6 is a partially enlarged view of Y in FIG. 5. Below, take the second part 12 provided with a first convex structure 122 and the housing 31 of the recording instrument provided with a second concave structure 311 as examples, so as to illustrate the technical solution of embodiments of this disclosure.

The inward convex part 121 includes a first inside surface 1211 which faces the first part 11. The first convex structure 122 includes a first convex rib 1221 which protrudes from the first inside surface 1211. The second concave structure 311 includes a first groove 3111 with an opening which faces the first inside surface 1211. The first convex rib 1221 is snapped into the first groove 3111.

Thus, considering that the first part 11 is located at the front surface of the host housing 100 as described above, which can also be understood as that the first inside surface 1211 is located behind the first part 11 and faces the first part 11, at this time, the housing 31 of the recording instrument is sandwiched between the first inside surface 1211 and the first part 11, and the first convex rib 1221 extends from left to right. Furthermore, the seal for the rear surface the housing 31 of the recording instrument can be achieved through the fitting of the first convex rib 1221 and the first groove 3111.

In addition, since the first convex rib 1221 is snapped into the first groove 3111, it can also form a first connective fixation between the second part 12 and the housing 31 of the recording instrument.

In order to improve the stability of the connective fixation between the housing 31 of the recording instrument and the second part 12, the housing 31 of the recording instrument is provided with a first through-hole 312 that connects the interior of the housing 31 of the recording instrument and the interior of the host housing 100. A first fastener 33 penetrates through the first through-hole 312. The inward convex part 121 is provided with a first fastening structure 123, and the first fastener 33 is detachably connected with the first fastening structure 123.

Specifically, the first fastener 33 can be a screw, pin, or the likes. For example, the first fastener 33 is a screw, and the first fastening structure 123 can have threaded holes to allow the screw to be threaded into the threaded hole after penetrating into the first through-hole 312.

It can also be understood that on the one hand, compared to that the first fastener 33 is exposed outside of the second part 12, since the first fastener 33 is hidden inside the housing 31 of the recording instrument and the host housing 100, the embodiment of this disclosure can make the outer surface of the second part 12 more tidy and beautiful. On the other hand, compared to that through holes are arranged at the second part 12 to assemble screws, the embodiment of this disclosure can avoid a decrease in seal performance caused by opening holes at the second part 12.

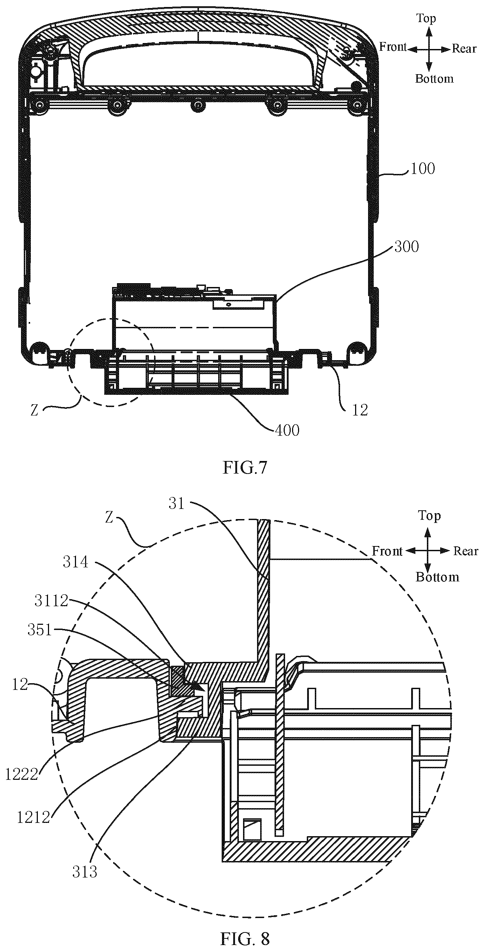

Please continue to refer to FIGS. 7 and 8. FIG. 7 is a structural diagram of a fitting structure between a recording instrument and a second part of the defibrillation device shown in FIG. 1. FIG. 8 is a partially enlarged view of Z in FIG. 7. The inward convex part 121 also includes two second inside surfaces 1212, and the housing 31 of the recording instrument is located between the two second inside surfaces 1212, which extend from different ends of the first inside surface 1211 toward the first part 11. Correspondingly, the first convex structure 122 also includes two second convex ribs 1222, each second inside surface 1212 is provided with one second convex rib 1222. The second concave structure 311 also includes two second grooves 3112, and one second convex rib 1222 is snapped into one second groove 3112.

At this point, combining that the first inside surface 1211 mentioned above is located behind the housing 31 of the recording instrument, it can be understood that one second inside surfaces 1212 is located at the left side of the housing 31 of the recording instrument and extends from rear to front, and the other second inside surface 1212 is located at the right side of the housing 31 of the recording instrument and extends from rear to front. Correspondingly, the second convex rib 1222 of each second inside surface 1212 extends from rear to front, so that the left and right sides of the housing 31 of the recording instrument are respectively fitted with one second inside surface 1212 to achieve a seal.

Below, we continue to provide examples of some optional structures of the housing 31 of the recording instrument to further explain and illustrate the second groove 3112 and the second convex rib 1222.

In some embodiments, two pairs of position-limiting ribs protrude from the outer surface of the housing 31 of the recording instrument, each pair of position-limiting ribs include a first position-limiting rib 313 and a second position-limiting rib 314. Both pairs of position-limiting ribs extend from the first inside surface 1211 toward the first part 11. Such that, it can be understood that one pair of position-limiting ribs protrude from rear to front at the left side of the housing 31 of the recording instrument, while the other one pair of position-limiting ribs protrude from rare to front at the right side of the housing 31 of the recording instrument. At this point, the first position-limiting rib 313 and the second position-limiting rib 314 in each pair of position-limiting ribs, as well as the outer surface of the housing 31 of the recording instrument, can form the second groove 3112 mentioned above for fitting with the corresponding second convex rib 1222.

Specifically, the first position-limiting rib 313 in the first pair of position-limiting ribs (such as the pair of position-limiting ribs located at the left side of the housing 31 of the recording instrument) abuts against a side of a first second convex rib 1222 (such as the second convex rib located at the left side of the housing 31 of the recording instrument), which side is adjacent to the outside of the second part 12. Optionally, it can also be understood as that the first position-limiting rib 313 abuts against a lower side of the second convex rib 1222. The second position-limiting rib 314 in the first pair of position-limiting ribs is arranged at one side of the first second convex rib 1222, which side back-faces the first position-limiting rib 313. Optionally, it can also be understood that the second position-limiting rib 314 is located above the corresponding second convex rib 1222. In this way, the second position-limiting rib 314 in the first pair of position-limiting ribs, the first second convex rib 1222, as well as the outer surface of the housing 31 of the recording instrument, together form a first insertion groove.

Correspondingly, the defibrillation device also includes a first insertion part 351, which is detachably inserted into a first insertion groove to clamp the second convex rib 1222 between the corresponding first insertion part 351 and the first position-limiting rib 313. Furthermore, the first second convex rib 1222 is tightly clamped between the first pair of position-limiting ribs through the first insertion part 351.

It can also be understood that since the first position-limiting rib 313 and the second position-limiting rib 314 extend from rear to front, one end of the first insertion groove which is adjacent to the first part 11, or in other words, a front end of the first insertion groove, is an open structure, so that the first insertion part 351 can be inserted into the first insertion groove from front to rear. In this way, during the assembly process of the housing 31 of the recording instrument, the first groove 3111 at the rear side of the housing 31 of the recording instrument can be tightly snapped with the first convex rib 1221 firstly; then the second convex rib 1222 at the left side of the housing 31 of the recording instrument can be inserted between the first position-limiting rib 313 and the second position-limiting rib 314 at the left side; and finally, the first insertion part 351 is inserted from front to rear into the first insertion groove, so that the second convex rib 1222 at the left side is tightly snapped into the first insertion groove.

Meanwhile, due to the addition of the first insertion part 351, the second convex rib 1222 at the left side can be more tightly snapped into the second groove 3112 at the left side of the housing 31 of the recording instrument. On the one hand, this can improve the seal effect of the seal structure at the left side of the housing 31 of the recording instrument, and on the other hand, can also improve the stability of the connection between the housing 31 of the recording instrument and the second part 12.

In addition, the first position-limiting rib 313 in the second pair (such as the pair of position-limiting ribs located at the right side of the housing 31 of the recording instrument) abuts against a side of a second second convex rib 1222 (such as the second convex rib located at the right side of the housing 31 of the recording instrument), which side is adjacent to the outside of the second part 12. Optionally, it can also be understood as that the first position-limiting rib 313 abuts against a lower side of the second convex rib 1222. The second position-limiting rib 314 in the second pair of position-limiting ribs is arranged at one side of the second second convex rib 1222, which side back-faces the first position-limiting rib 313. Or in other words, the second position-limiting rib 314 is located above the corresponding second convex rib 1222. In this way, the second position-limiting rib 314 in the second pair of position-limiting ribs, the second second convex rib 1222, as well as the outer surface of the housing 31 of the recording instrument, together form a second insertion groove.

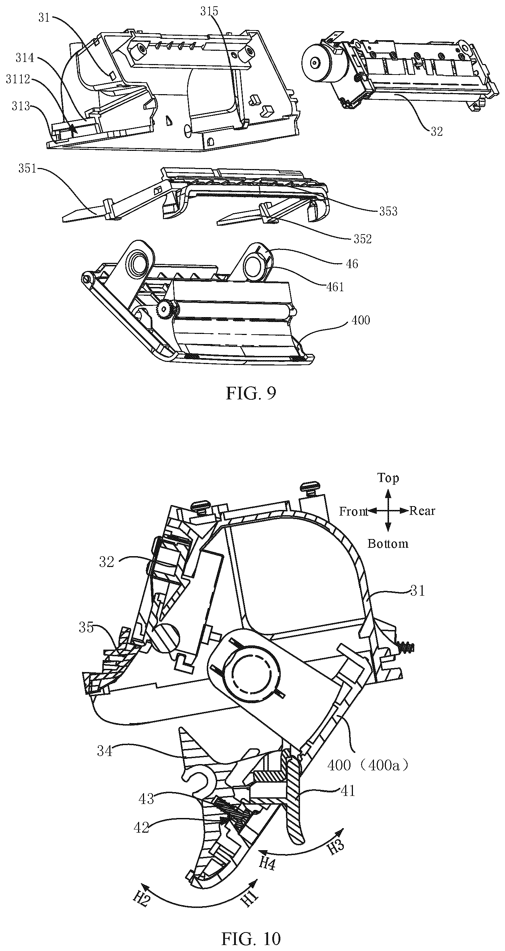

Correspondingly, please continue to combine FIG. 9, which is an exploded view of a recording instrument and a door body of the defibrillation device shown in FIG. 1. The defibrillation device also includes a second insertion part 352, which can be detachably inserted into the second insertion groove to clamp the second convex rib 1222 between the corresponding insertion part and the first position-limiting rib 313. Furthermore, the second convex rib 1222 is clamped between the second pair of position-limiting ribs through the second insertion part 352.

It can also be understood that since the first position-limiting rib 313 and the second position-limiting rib 314 extend from rear to front, one end of the second insertion groove which is adjacent to the first part 11, or in other words, a front end of the second insertion groove, is an open structure, so that the second insertion part 352 can be inserted into the second insertion groove from front to rear. In this way, during the assembly process of the housing 31 of the recording instrument, the first groove 3111 at the rear side of the housing 31 of the recording instrument can be tightly snapped with the first convex rib 1221 of the first inside surface 1211 firstly; then the second convex rib 1222 at the right side of the housing 31 of the recording instrument can be inserted between the first position-limiting rib 313 and the second position-limiting rib 314 at the right side; and finally, the second insertion part 352 is inserted from front to rear into the second insertion groove, so that the second convex rib 1222 at the right side is tightly snapped into the second insertion groove.

Meanwhile, due to the addition of the second insertion part 352, the second convex rib 1222 at the right side can be more tightly snapped into the second groove 3112 at the right side of the housing 31 of the recording instrument. On the one hand, this can improve the seal effect of the seal structure at the right side of the housing 31 of the recording instrument, and on the other hand, can also improve the stability of the connection between the housing 31 of the recording instrument and the second part 12.

In some embodiments, the defibrillation device further includes a connection part 353, which is partially located between the housing 31 of the recording instrument and the first part 11. Moreover, the connection part 353 is located between two second inside surfaces 1212. Ends of the connection part 353, which are respectively adjacent to each second inside surface 1212, are respectively connected with one insertion part to enable both ends of the connection part 353 to clamp the housing 31 of the recording instrument through the insertion part.

Specifically, the connection part 353 is connected with the first insertion part 351 at one end, which end is adjacent to a first second inside surface 1212 (such as the second inside surface 1212 at the left side), and the connection part 353 is connected with the second insertion part 352 at one end, which end is adjacent to the other second inside surface 1212 (such as the second inside surface 1212 at the right side). Furthermore, during the assembly of the housing 31 of the recording instrument, the connection part 353 can be pulled and pushed, so as to simultaneously insert the first insertion part 351 into the first insertion groove and insert the second insertion part 352 into the second insertion groove, ultimately improving the disassembling efficiency of the housing 31 of the recording instrument.

In some embodiments, the connection part 353 and at least one insertion part can be integrally formed, thereby reducing the overall number of components of the defibrillation device, thus further reducing the assembly difficulty of the defibrillation device and improving the assembly efficiency of the defibrillation device. Of course, the connection strength between the connection part 353 and the insertion part can also be improved. For example, the first insertion part 351, the second insertion part 352, and the connection part 353 are integrally formed through injection molding, or only the connection part 353 and the first insertion part 351 are integrally formed. Of course, in some other embodiments, the connection part 353 and the both insertion parts can be individually manufactured and assembled, and embodiments of this disclosure do not limit this.

Below, we continue to provide examples of the seal structure between the front side of the housing 31 of the recording instrument and the first part 11 in the embodiment of this disclosure, in conjunction with the structure of the connection part 353.

Please refer to FIG. 3. A side of the connection part 353, which side back-faces the first inside surface 1211 is provided with a third convex structure or a third concave structure 3531. Optionally, in other words, a front side of the connection part 353 is provided with a third convex structure or a third concave structure 3531. The inner surface of the first part 11 is provided with a fourth concave structure or a fourth convex structure 111 that fits with the third convex structure or the third concave structure 3531 to seal between the connection part 353 and the first part 11. Moreover, the front side of the housing 31 of the recording instrument abuts against so as to be fixed to the inside surface of the first part 11 through the connection part 353. In this way, the seal between the connection part 353 and the first part 11 can also seal between the first part 11 and the front side of the housing 31 of the recording instrument.

Specifically, it can be that the connection part 353 is provided with a third convex structure, the inner surface of the first part 11 is provided with a fourth concave structure. Optionally, it can be that the connection part 353 is provided with a third concave structure 3531, and the inner surface of the first part 11 is provided with a fourth convex structure 111. This embodiment of this disclosure does not limit this.

Of course, in some other embodiments, the connection part 353 can also be provided with both the third convex structure and the third concave structure 3531, and the first part 11 can be provided with both the fourth concave structure and the fourth convex structure 111. Furthermore, the third convex structure and the fourth concave structure can be fitted to form a first seal between the connection part 353 and the first part 11, while the third concave structure 3531 and the fourth convex structure 111 can be fitted to form a second seal between the connection part 353 and the first part 11. The embodiments of this disclosure make no limitation for this.

In summary, the rear side of the housing 31 of the recording instrument is sealed by the fitting of the first groove 3111 and the first convex rib 1221. The left and right sides of the housing 31 of the recording instrument can be sealed by the fitting of the second groove 3112 and the second convex rib 1222. The front side of the housing 31 of the recording instrument can be sealed by the fitting of the connection part 353 and the first part 11.

Optionally, one or more sides of the front side, rear side, left side, and right side of the housing 31 of the recording instrument can be sealed through corresponding concave and convex structures, while the remaining portions can be sealed through other structures, such as sealing rubber strips, sealing sponges, etc. Embodiments of this disclosure do not limit this.

The above are some examples of the seal structures at the front, left, right, and rear sides of the housing 31 of the recording instrument in the embodiment of this disclosure. The following continues to provide examples of a structure of the door body 400 in the embodiment of this disclosure.

Please continue to refer to FIG. 10, which is a cross-sectional view of a recording instrument and a door body shown in FIG. 4. The movable connection methods of the door body 400 can be diverse, for example the door body 400 can be rotatably connected or movable connected, and embodiments of this disclosure do not limit this.

For example, at least one of the housing 31 of the recording instrument and the host housing 100 is rotatably connected with the door body 400 to enable the door body 400 to rotate in a first direction H1, so as to open the housing 31 of the recording instrument, and to enable the door body 400 to rotate in a second direction H2, so as to close the housing 31 of the recording instrument. The first direction **H1** is opposite to the second direction H2, so it can also be understood that the door body 400 can rotate forward to open the door and backward to close the door.

Wherein, the door body 400 can only be rotatably connected with the housing 31 of the recording instrument, so it can be considered as a part of the recording instrument 300 for synchronous disassembly and assembly with the recording instrument 300. Alternatively, the door body 400 can also be rotatably connected only to the host housing 100, or the door body 400 can be rotatably connected with both the host housing 100 and the recording instrument 300 at the same time. Embodiments of this disclosure do not limit this.

In some embodiments, in order to facilitate the opening and closing of the door body 400, the door body 400 is provided with a handle 41. A fixed end of the handle 41 is rotatably connected with the door body 400, so that handle 41 can rotate in a third direction H3 until a free end of the handle 41 is away from the door body 400, and the handle 41 can rotate in a fourth direction H4 until the free end of the handle 41 is adjacent to the door body 400, wherein the third direction H3 is opposite to the fourth direction H4. Furthermore, the handle 41 is capable of rotating in the fourth direction H4 to be attached to the door body 400 for easy storage, and the handle 41 is further capable of rotating in the third direction H3 to stand up from the door body 400 for facilitating grip of user.

Wherein, the third direction H3 can be same as the first direction H1, and the fourth direction H4 can be same as the second direction H2.

It can be understood that taking the second part 12 as a bottom wall of the host housing 100 as an example, the fixed end of the door body 400 is located at its rear side and is rotatably connected with the housing 31 of the recording instrument in a closed state. At this point, if the door body 400a of the recording instrument rotates in the first direction H1 to open the door, the free end of the door body 400, or in other words, the front end of the door body 400, flips backwards. If the handle 41 can rotate in the third direction H3 until the free end of the handle 41 is far away from the door body 400, the free end of the handle 41 flips backwards to stand up from the door body 400, so as to be held. Therefore, the user can flip backward the handle 41 to grasp it and then continue to flip the handle 41 backward to open the door body 400. Therefore, compared to pulling the handle 41 forward, leftward, or rightward firstly, and then backwards to open the door, the door body 400 of embodiments of this disclosure is more convenient to open.

Alternatively, the third direction H3 can also be opposite to the first direction H1, and the fourth direction H4 can also be opposite to the second direction H2. The embodiment of this disclosure does not limit this.

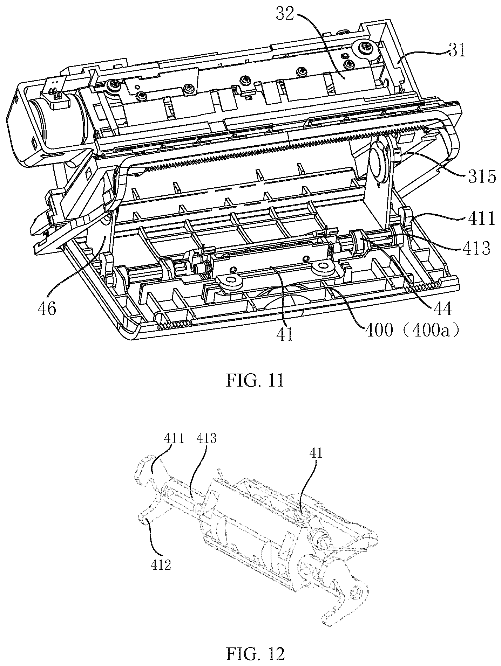

Please continue to refer to FIG. 11, which is a first assembly diagram of a recording instrument and a door body of the defibrillation device shown in FIG. 1. The inner wall of the housing 31 of the recording instrument is provided with a first snap-fit 315, and the handle 41 is provided with a second snap-fit 411. When the door body 400 closes the housing 31 of the recording instrument, the handle 41 is capable of rotating in the fourth direction H4 until the first snap-fit 315 is snap-fitted with the second snap-fit 411, so as to lock the door body 400 and the housing 31 of the recording instrument. Moreover, the handle 41 is capable of rotating in the third direction H3 to separate the first snap-fit 315 and the second snap-fit 411, so as to unlock the door body 400 and the housing 31 of the recording instrument.

That is to say, when the door body 400 closes the housing 31 of the recording instrument, the handle 41 rotates in the fourth direction H4 to be attached to the door body 400, and the first snap-fit 315 is also snap-fitted with the second snap-fit 411 to lock the door body 400. When the handle 41 rotates in the third direction H3 to stand up from the door body 400, the first snap-fit 315 is also separated from the second snap-fit 411 to unlock the door body 400. Such that, when the user opens the door, the user can pull back the handle 41 to complete three actions in one go, which actions include pulling out the handle 41, unlocking the second snap-fit 411, and opening the door body 400. In this way, the opening of the door body 400 is more convenient.

Meanwhile, since the first snap-fit 315 is assembled inside the housing 31 of the recording instrument, compared to exposing the first snap-fit 315, the embodiment of this disclosure can also make the external surface of the defibrillation device more beautiful, tidy, and not easily damaged.

Please continue to refer to FIG. 12, which is a structural diagram of a handle shown in FIG. 11. The handle 41 can also be provided with an abutting part 412. The abutting part 412 is located at a side of the fixed end of the handle 41, which side back-faces the free end of the handle 41, and a distance from the fixed end of the handle 41 to the abutting part is smaller than a distance from the fixed end of the handle 41 to the free end of the handle 41. Furthermore, the handle 41 can be regarded as a lever as a whole, with the fixed end of the handle 41 being a fulcrum of the handle 41, and a first force arm formed from the abutting part to the fulcrum is smaller than a second arm formed from the free end of the handle 41 to the fulcrum. at least at one moment during handle 41 rotating in the third direction H3, the abutting part 412 abuts against the housing 31 of the recording instrument. That is to say, during the process of rotating the handle 41 to stand up relative to the door body 400, the first force arm can form a resistance arm, and the second force arm can form a power arm. Since the power arm is larger than the resistance arm, user can open the door through the handle 41 more effortlessly.

Specifically, the abutting part 412 can be rotated to abut against the first snap-fit 315. Furthermore, there is no need to assemble additional structures inside the housing 31 of the recording instrument to fit the abutting part 412, such that the internal structure of the housing 31 of the recording instrument can be simpler, reducing the manufacturing difficulty and support cost of the housing 31 of the recording instrument.

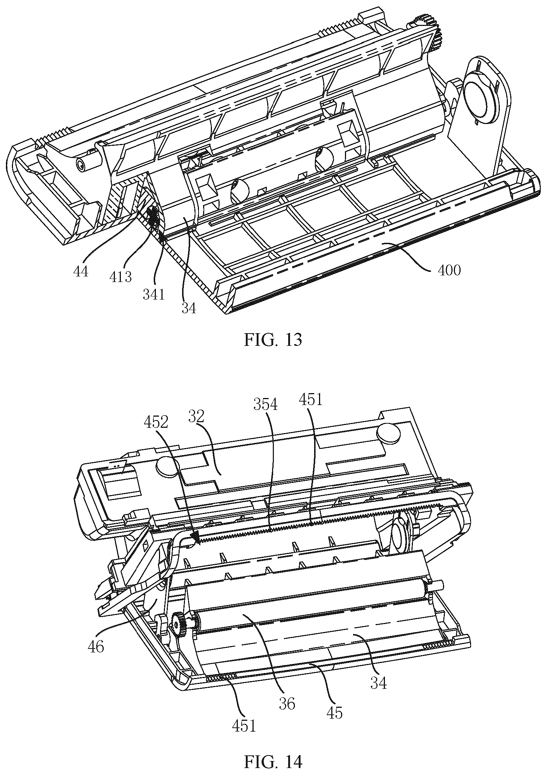

Please continue to refer to FIG. 13, which is a partial cross-sectional view of a handle and a door body shown in FIG. 10. In order to ensure a smoother output process of printing paper to the outside of the host housing 100, the inner surface of the door body 400 can be provided with a first guide component 34. The first guide component 34 is configured to guide the printing paper inside the housing 31 of the recording instrument to the outside of the host housing 100.

Continuing to combine the above embodiment in which the door body 400 is provided with the handle 41, the fixed end of the handle 41 can be provided with a rotating shaft 413, so that the handle 41 can rotate the door body 400 through the rotating shaft 413. Specifically, the first guide component 34 is provided with a third groove 341 at a side which faces the door body 400, wherein the third groove 341 and the inner surface of the door body 400 forms a rotating hole. The rotating shaft 413 is rotatably assembled inside the rotating hole. Therefore, the first guide component 34 can also be reused for assembling and fixing the handle 41 to reduce the overall number of components in the defibrillation device, thereby making the defibrillation device lighter and thinner, and making disassembly and assembly easier.

Wherein, the second snap-fit 411 and the abutting part 412 mentioned above can be formed at the rotating shaft 413.

Wherein, in order to drive the handle 41 to rotate in the fourth direction H4, a torsion spring can also be provided at the handle 41. One torsion arm of the torsion spring abuts against the inner surface of the door body 400, and the other torsion arm of the torsion spring abuts against the rotating shaft 413, so as to drive the rotating shaft 413 and the handle 41 connected with the rotating shaft 413 to rotate in the fourth direction H4, thus ultimately enabling the handle 41 to be attached to the door body 400 and enabling the first snap-fit 315 to be snap-fitted with the second snap-fit 411.

The handle 41 and the rotating shaft 413 can be integrally formed, or detachable connected through screw connection, sleeve connection, fitting connection, etc. The embodiment of this disclosure does not limit this.

In some embodiments, as shown in FIG. 10, the door body 400 may be provided with a second through-hole 42, a second fastener 43 penetrates through and is arranged inside the second through-hole 42. The second fastener 43 is detachably connected with the first guide component 34, and then the first guide component 34 is fixed and assembled through the second fastener 43.

Specifically, the second fastener 43 can be a screw, pin, or the like. Taking the second fastener 43 as an example, which is a screw, the first guide component 34 can have a threaded hole, so that the screw can be threaded into the threaded hole after penetrating the second through hole 42.

In order to enhance the appearance of the defibrillation device, the handle 41 can be rotated in the fourth direction H4 to cover the second through-hole 42. Furthermore, when the door body 400 closes the housing 31 of the recording instrument, the handle 41 can rotate in the fourth direction H4 to be attached to the door body 400, so as to cover the second through hole 42 and the second fastener 43 penetrating the second through hole 42.

In some embodiments, as shown in FIGS. 11 or 13, the door body 400 may also be provided with a clamping part 44, and the rotating shaft 413 can be rotatably clamped by the clamping part 44. Furthermore, the assembly and fixation of the rotating shaft 413 can be achieved through the clamping part 44.

It can be understood that the rotating shaft 413 can be assembled through the clamping part 44, and the rotating shaft 413 can also be assembled through the first guiding component 34. Embodiments of this disclosure do not limit this.

Optionally, the rotating shaft 413 can also be assembled simultaneously through the clamping part 44 and the first guide component 34 to ensure the assembly strength of the rotating shaft 413 through a dual fixation structure. Such that, the second snap-fit 411, which is arranged at the rotating shaft 413, can be prevented from being unable to lock the door body 400 due to the damage to the connection structure of the rotating shaft 413.

For example, the handle 41 can be arranged in a middle position of the door body 400, so that the corresponding second fastener 43 is also located inside the middle position of the door body 400 to fix the first guide component 34. At this time, at least one clamping part 44 is arranged at both left and right ends of the inner surface of the door body 400.

Of course, in some embodiments, the clamping part 44 can also be clamped at any position along an axial direction of the rotating shaft 413, and embodiments of this disclosure do not limit this. It can also be understood that during the specific assembly process, the rotating shaft 413 of the handle 41 can be clamped by the clamping part 44 to achieve preliminary assembly and fixation, and then the first guide component 34 can be assembled at the inner surface of door body 400 to further tighten the handle 41. From this, it can be seen that the embodiment of this disclosure enables the handle 41 to have the advantages of convenient and reliable assembly by double fixing the rotating shaft 413 with the clamping part 44 and the first guide component 34.

The above are some examples of the opening method of the door body 400 in the embodiment of this disclosure. The following continues to provide further explanation and illustration of a structure related to the printing paper in the recording instrument 300 in conjunction with the first guide component 34.

The inner surface of the door body 400 is provided with a first guide component 34, and the housing 31 of the recording instrument is provided with a second guide component 35 that fits with the first guide component 34. When the door body 400 is rotated to close the housing 31 of the recording instrument, the first guide component 34 and the second guide component 35 together form a paper output channel, which channel is configured to guide the printing paper inside the housing 31 of the recording instrument to be outputted to the outside of the host housing 100.

On the one hand, since the first guide component 34 can rotate with the door body 400, during the process of replacing the printing paper, some of the printing paper is directly pulled out a roll of the printing paper and placed at the first guide component 34. After the door body 400 is rotated to close the housing 31 of the recording instrument, the printing paper is directly located inside the paper output channel. In this way, during the process of replacing the printing paper, there is no need to manually pull the printing paper through different guide components or zigzagged paper output channels, making the replacement of the printing paper more convenient.

On the other hand, since the printing component 32 is at least partially located at the rear side of the display component 200 in the embodiment of this disclosure, compared to that the printing component 32 and the display component 200 are arranged side by side at the surface side of the first part 11, the printing component 32 is naturally located in a deeper position of the host housing 100, which results in that the printing paper needs to take a longer path to be outputted to the front side of the host housing 100 after being printed by the printed component 32. Therefore, the possibility of jam for the printing paper inside the host housing 100 is even greater. Therefore, the embodiment of this disclosure can reduce the possibility of jam for the printing paper inside the host housing 100 by setting an output channel to guide the printing paper, thereby improving the reliability of the defibrillation device.

It can also be understood that the first insertion part 351, the second insertion part 352, and the connection part 353 mentioned above can all be portions of the second guide component 35, and the housing 31 of the recording instrument can be sealed by reusing the second guide component 35. Of course, the second guiding component 35 can also be an independent component fixed to the above-mentioned connection part 353, and embodiments of this disclosure do not limit this.

In some embodiments, please continue to refer to FIG. 14, which is a second assembly diagram of a recording instrument and a door body of the defibrillation device shown in FIG. 1. The second guide component 35 is provided with a first paper output surface 354 at one end, which end is adjacent to the first part 11, and the door body 400 includes a second paper output surface 45 that is fitted with the first paper output surface 354. When the door body 400 is rotated to close the housing 31 of the recording instrument, the first paper output surface 354 and the second paper output surface 45 form a paper output opening 425, so as to output the printing paper in the paper output channel from the paper output opening 452 to the outside of the host housing 100. Furthermore, during the process of replacing the printing paper, some of the printing paper is directly pulled out from a roll of the printing paper, and placed at the first guide component 34 and the free end of the door body 400. After the door body 400 is rotated to close the housing 31 of the recording instrument, the printing paper is directly located inside the paper output channel. In this way, during the process of replacing the printing paper, there is no need to manually pull the printing paper through different guide components or zigzagged paper output channels, which make the replacement of printing paper more convenient.

Wherein, in order to facilitate the user to tear off the printing paper, at least one of the first paper output surface 354 and the second paper output surface 45 is provided with a paper tearing structure 451. That is to say, it can be that only the first paper output surface 354 can be provided with a paper tearing structure 451. It can also be that only the second paper output surface 45 is provided with a paper tearing structure 451. It can also be that both the first paper output surface 354 and the second paper output surface 45 are provided with a paper tearing structure 451, allowing user to tear paper from more directions.

In some embodiments, at least one end of the second paper output surface 45 is provided with a concave-convex structure along a width direction of outputted printing paper to form the paper tearing structure 451, and the middle portion of the second paper output surface 45 along the width direction of the outputted printing paper is a plane. Accordingly, the friction between the middle portion of the second paper output surface 45 and the printing paper is smaller to reduce the probability of jam for the printing paper at the paper output opening 452. At the same time, the paper tearing structures 451 at both ends enable edges of the printing paper to be more easily torn out, so as to facilitate tearing off the printing paper.

Alternatively, it is possible that the end of the paper output channel, which is adjacent to the first part 11, is the paper output opening 452. Therefore, the printing paper can be directly outputted to one side of the first part 11 of the host housing 100, or in other words, directly outputted to the front side of the host housing 100 through the paper output channel.

Wherein, in order to facilitate the user to tear off the printing paper, the second guide component 35 and/or the first guide component 34 are provided with the paper tearing structure 451 at the paper output opening 452. That is to say, it can be that only the second guide component 35 is provided with the paper tearing structure 451 at the paper output opening 452. It can also be that only the first guide component 34 is provided with the paper tearing structure 451 at the paper output opening 452. It can also be that the first guide component 34 and the second guide component 35 are both provided with the paper tearing structure 451 at the paper output opening 452, allowing user to tear paper from more directions.

As an example, the first guide component 34 is provided with a third paper output surface at the paper output opening 452. At least one end of the third paper output surface is provided with a concave-convex structure along the width direction of the outputted printing paper to form the tearing structure 451, and the middle portion of the third paper output surface along the width direction of the outputted printing paper is a plane. Accordingly, the friction between the middle portion of the paper output surface and the printing paper is smaller to reduce the probability of jam for the printing paper at the paper output opening 452. At the same time, the paper tearing structures 451 at both ends enable edges of the printing paper to be more easily torn out, so as to facilitate tearing off the printing paper.

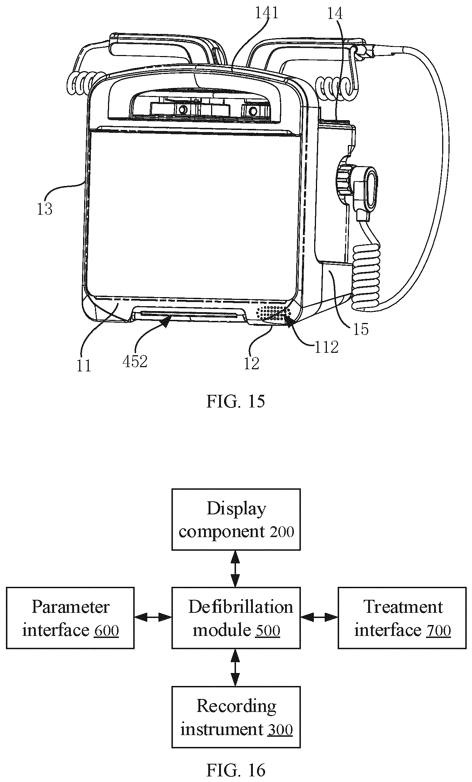

Please continue to refer to FIG. 15, which is a 3D diagram of the defibrillation device shown in FIG. 1. In order to enable a larger display component 200 to be assembled to the first part 11, the first part 11 further includes a sound output opening 112, which is arranged side by side with the paper output opening 452 at the same side of the display component 200. For example, the paper output opening 452 and the sound output opening can be arranged side by side at the lower side of the first part 11. Compared to arranging the paper output opening 452 at the lower side of the first part 11 and arranging the sound output opening 112 at the left side of the first part 11, a distance between the left side of the first part 11 and the display component 200 in embodiments of this disclosure can be made smaller, which enables the width of the display component 200 to be larger in the left-right direction, so as to increase the screen-to-body ratio of the display component 200. Of course, in some other embodiments, the sound output opening 112 and the paper output opening 452 can also be respectively arranged at different sides of the first part 11, and embodiments of this disclosure do not limit this.

In order to make the printing paper move more smoothly in the paper output channel, the first guide component 34 includes at least one smooth surface, which is configured to form an inner surface of the paper output channel to support the printing paper. Furthermore, by reducing the friction between the printing paper and the first guide component 34 through the smooth surface, the probability of jam for the printing paper in the output channel is reduced.

It can be understood that there are one or more smooth surfaces, and the embodiments of this disclosure make no limitation for this. Specifically, a moving path of the printing paper can be determined based on the placement position of the printing paper in the housing 31 of the recording instrument, the printing position of the printing component 32, and the position of the paper output opening 452, etc., and then the printing paper can be supported and guided through one or more smooth surfaces along the movement path of the printing paper.

In some embodiments, in order to facilitate the printing of the printing paper by the printing component 32, the recording instrument 300 may also include a paper roller 36, which is configured to fit with the printing component 32 to press the printing paper tightly onto the printing position of the printing component 32.

For example, as shown in FIG. 14, the paper roller 36 is rotatably assembled at the first guide component 34 to support the printing paper passing through the first guide component 34, and the printing component 32 is configured to press and print the printing paper supported by the paper roller 36.

Alternatively, the paper roller 36 can also be rotatably assembled at the housing 31 of the recording instrument or the second guide component 35, the printing component 32 is assembled at the first guide component 34, and the paper roller 36 is configured to press the printing paper passing through the first guide component 34 onto the printing component 32 for printing by the printing component 32.

It can be understood that since one of the printing component 32 and the paper roller 36 is assembled at the first guide component 34, while the first guide component 34 is assembled at the door body 400 and can be opened by following the rotation of the door body 400, such that during the process of replacing the printing paper, some printing paper is directly pulled out from the roll of printing paper and placed at the first guide component 34. In this way, after the door body 400 is rotated to close the housing 31 of the recording instrument, the printing paper is directly located inside the paper output channel and clamped between the printing component 32 and the paper roller 36. Such that, during the process of replacing the printing paper, there is no need to manually pull the printing paper through different guide components or zigzagged paper output channels, making the replacement of the printing paper more convenient.

Wherein, the printing component 32 can be a thermal printing device, an ink printing device, etc., which is not limited by the embodiments of this disclosure.

**In** some embodiments, in order to achieve the automatic paper output function of the recording instrument 300, the paper roller 36 may be provided with a first transmission structure, the printing component 32 may be provided with a driving component, which is connected with a second transmission structure. The second transmission structure and the first transmission structure can be separated and connected with each other, so that when the door body 400 opens the housing 31 of the recording instrument, the first transmission structure and the second transmission structure can be separated, and when the door body 400 closes the housing 31 of the recording instrument, the first transmission structure and the second transmission structure can be docked to form transmission connection.

For example, the driving components can be electric machines, motors, etc. The first transmission structure can be a first gear arranged at an axial end of the paper roller 36, and the second transmission structure can be a second gear connected with the driving component. When the door body 400 closes the housing 31 of the recording instrument, the first gear and the second gear mesh to achieve transmission connection. Of course, the first transmission structure and the second transmission structure can also be mutually fitted gears and racks, and the embodiments of this disclosure make no limitation for this.

The fixation methods of the printing paper accommodated inside the housing 31 of the recording instrument can be diverse, for example the printing paper can be fixed at the door body 400 or fixed at the housing 31 of the recording instrument. Embodiments of this disclosure do not limit this.

For example, the printing paper is fixed at the door body 400. The inner surface of the door body 400 is provided with a fixing structure 46 for the printing paper, which is used to assemble the printing paper. It can be understood that since the fixing structure 46 for the printing paper can follow the movement of the door body 400 to the outside of the housing 31 of the recording instrument when opening the door body 400, replacing the printing paper at the door body 400 is more convenient and simpler compared to replacing the printing paper inside the housing 31 of the recording instrument.

For example, as shown in FIG. 9, the fixing structure 46 for the printing paper may include two spaced elastic clamping arms to clamp the roll made of printing paper.

Specifically, the roll of printing paper can include a hollow paper drum and printing paper wound outside the drum. In this way, a circular lug boss 461 can be arranged at a side of each elastic clamping arm, which side faces the other elastic clamping arm, and the circular lug boss 461 of the two elastic clamping arms can be pivoted to fix the paper drum of the roll of printing paper.

The above are some examples of the structure of the recording instrument 300 in the embodiment of this disclosure. Below, we continue to provide examples of the overall structure of the defibrillation device in the embodiment of this disclosure.

Please refer to both FIG. 15 and FIG. 16. FIG. 16 is a block diagram of the defibrillation device shown in FIG. 15. The host housing 100 also includes a third part 13, a fourth part 14, and a fifth part 15. The first part 11, the second part 12, the third part 13, the fourth part 14, and the fifth part 15 face different sides, respectively.

For example, in combination with that the first part 11 located at the front side of the host housing 100, the second part 12, the third part 13, the fourth part 14, and the fifth part 15 can be sequentially located at the bottom side, left side, top side, and right side of the host housing 100. In other words, the second part 12, the third part 13, the fourth part 14, and the fifth part 15 can be sequentially the bottom wall, left wall, top wall and right wall of the host housing 100. Of course, it can also be understood that the orientations of each part at the host casing 100 are interchangeable, and the embodiments of this disclosure make no limitation for this.

The defibrillation device can also include a defibrillation module 500, a parameter interface 600, and a treatment interface 700. The defibrillation module 500 is assembled inside the host housing 100 to process state information of human body and provide a defibrillation current and/or voltage. The parameter interface 600 is arranged inside the third part 13, and is configured to connect detection components, such as some sensors or detection electrodes. The parameter interface 600 is also electrically connected with the defibrillation module 500 to enable the defibrillation module 500 to obtain state information of human body through the detection components, thereby providing more suitable defibrillation current and/or voltage. The treatment interface 700 is arranged inside the fifth part 15, and is configured to connect a defibrillation electrode. The treatment interface 700 is also electrically connected with the defibrillation module 500 to enable the defibrillation module 500 to apply the defibrillation current and/or voltage to the human body through the defibrillation electrode.

It can be understood that the defibrillation electrode can be a pre-assembled component of the defibrillation device, or be an external or consumable component which is plugged into the treatment interface 700, etc. The embodiment of this disclosure does not limit this.

The defibrillation electrode can be either an electrode sheet or an electrode plate. Correspondingly, taking the fourth part 14 located at the top surface of the host housing 100 as an example, a storage area can be arranged at the fourth part 14 for placing defibrillation electrodes, such as electrode sheets or electrode plates.

In order to facilitate the use to move the defibrillation device, the defibrillation device can also include a holding structure 141, which is arranged inside the fourth part 14. Specifically, the holding structure 141 can be a handle or the likes to facilitate the user to lift the defibrillation device.

The display component 200 can also be electrically connected with the defibrillation module 500, so as to display the working state of the defibrillation device, state information of patient body, usage indication, and so on. Of course, the display component 200 can also be a touch screen with touch function for user to interact with the defibrillation device, which is not limited in embodiments of this disclosure.

The recording instrument 300 can also be electrically connected with the defibrillation module 500 for printing work log of the defibrillation device, state information of patient body, and so on.

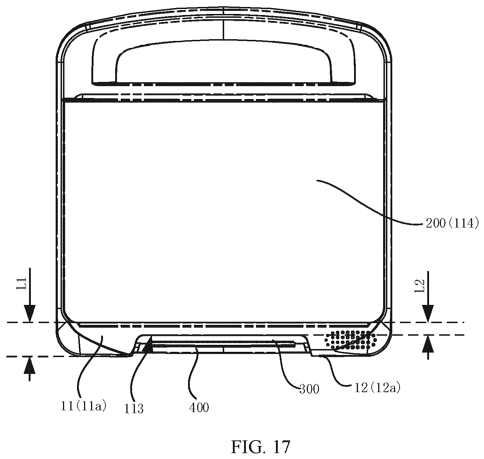

Please continue to refer to FIG. 17, which is another front view of the defibrillation device shown in FIG. 1. The embodiment of this disclosure also provides a defibrillation device, which includes a host housing 100, a display component 200, and a recording instrument 300.

The host housing 100 serves as a housing of the entire defibrillation device, and includes a first part **11** at a first side and a second part 12 at a second side. That is to say, assuming that the first part 11 is located at a front side of the host housing 100, or in other words that the first part 11 is a front wall of the host housing 100, the second part 12 can be located at a bottom surface, a left surface, a right surface, a rear surface, or a top surface of the host housing 100, or in other words, the second part 12 can be a bottom wall, a left wall, a right wall, a rear wall, or a top wall of the host housing 100. The embodiments of this disclosure make no limitation for this. In the following, the embodiment of this disclosure is explained by taking the first part 11 located at the front surface of the host housing 100 as an example.

The display component 200 is assembled at the first part 11, which can be understood as being assembled at the front surface or front wall of the host housing 100.

The recording instrument 300 is at least partially assembled at the host housing 100, and is configured to hold and print the printing paper.

Wherein, the defibrillation device is configured such that: the printing paper which is to be printed is capable of being placed into and taken out from the recording instrument via the second side, which means that the defibrillation device is configured such that, the printing paper is replaced from the area outside the front surface of the host housing 100. Therefore, compared to reserving a larger area at the front surface of the host housing 100 for paper replacement, the display component 200 at the front surface of the host housing 100 in embodiments of this disclosure can be made larger to increase the screen-to-body ratio at the front surface of the host housing 100.

The defibrillation device is also configured such that, the printed paper is outputted from the first side, which means that the recording instrument 300 can output the printed paper from the front surface of the host housing 100 for facilitating the user to obtain.

Meanwhile, as there is no need to reserve a large area at the front surface of the host housing 100 for paper replacement, a distance L1 between a frame of display component 200, which frame is adjacent to the second part 12, and the second part 12 is less than 35 millimeters.

For example, the second part 12 can be located at the bottom surface of the host housing 100, which means that the distance L1 between a lower frame of the display component 200 and the second part 12 can be 35 millimeters, 24 millimeters, 12.3 millimeters, etc. The printing paper can be outputted from an area, which is less than 35 millimeters below the display component 200, so that the screen-to-body ratio of the display component 200 at the front surface of the host housing 100 can be larger.

In some embodiments, the structures of the host housing 100, display component 200, and recording instrument 300 can respectively refer to in the aforementioned host housing 100, display component 200, and recording instrument 300. The embodiments of this disclosure give no unnecessary details .

In some embodiments, the defibrillation device may further include one or more of the door body 400, the first insertion part 351, the second insertion part 352, the connection part 353, the first guide component 34, the second guide component 35, the defibrillation module 500, the parameter interface 600, the holding structure 141, and the treatment interface 700. Wherein the specific structures of the door body 400, the first insertion part 351, the second insertion part 352, the connection part 353, the first guide component 34, the second guide component 35, the defibrillation module 500, the parameter interface 600, the holding structure 141, and the treatment interface 700, may refer to the above mentioned door body 400, first insertion part 351, second insertion part 352, connection part 353, first guide component 34, second guide component 35, defibrillation module 500, parameter interface 600, holding structure 141, and treatment interface 700, respectively. The embodiments of this disclosure give no unnecessary details herein.

As shown in FIG. 17, embodiments of this disclosure also provide a defibrillation device, which includes a host housing 100, a display component 200, and a recording instrument 300.

The host housing 100 can serve as a housing of the entire defibrillation device, and includes a first part 11 at a first side and a second part 12 at a second side. That is to say, assuming that the first part 11 is located at the front side of the host housing 100, or in other words that the first part 11 is a front wall of the host housing 100, the second part 12 can be located at a bottom surface, a left surface, a right surface, a rear surface, or a top surface of the host housing 100, or that the second part 12 can be the a bottom wall, a left wall, a right wall, a rear wall, or a top wall of the host housing 100. The embodiments of this disclosure make no limitation for this. In the following, the embodiment of this disclosure is explained by taking the first part 11 located at the front surface of the host housing 100 as an example.

The display component 200 can be assembled at the first part 11, which can be understood as being assembled at the front surface of the host housing 100.

The recording instrument 300 is at least partially assembled at the host housing 100, and is configured to hold and print the printing paper.

Wherein, the defibrillation device is configured such that: the printing paper which is to be printed is capable of being placed into and taken out from the recording instrument via the second side, which means that the defibrillation device is configured such that, the printing paper is replaced from the area outside the front surface of the host housing 100. Therefore, compared to reserving a larger area at the front surface of the host housing 100 for paper replacement, the display component 200 at the front surface of the host housing 100 in embodiments of this disclosure can be made larger to increase the screen-to-body ratio at the front surface of the host housing 100.

The first part 11 includes a first side edge, which located in a fifth direction of the display component 200, wherein the first side edge is provided with a first opening 11 to output printed paper. This means that the recording instrument 300 can output the printed paper from the front surface of the host housing 100 for facilitating user to obtain. An orthographic projection of the first opening 113 in the fifth direction at least partially overlaps with an orthographic projection of the display component 200 in the fifth direction, or the first opening 113 is at least partially located at the lower side of the display component 200. A minimum distance L2 between the display component 200 and the first opening 113 is less than 25 millimeters, for example, the minimum distance L2 can be 25 millimeters, 20 millimeters, 13.7 millimeters, 8.1 millimeters, and so on. Furthermore, by transferring a paper replacement area for the recording instrument 300, which is reserved by the host housing 100, to other surfaces of the host housing 100, and compressing the distance between the display component 200 and the first opening 113, the display component 200 has a larger screen proportion at the front surface of the host housing 100.

In some embodiments, the structures of the host housing 100, the display component 200, and the recording instrument 300 can respectively refer to the aforementioned host housing 100, display component 200, and recording instrument 300. The embodiments of this disclosure give no unnecessary details .

In some embodiments, the defibrillation device may further include one or more of the door body 400, the first insertion part 351, the second insertion part 352, the connection part 353, the first guide component 34, the second guide component 35, the defibrillation module 500, the parameter interface 600, the holding structure 141, the treatment interface 700. The specific structures of the door body 400, the first insertion part 351, the second insertion part 352, the connection part 353, the first guide component 34, the second guide component 35, the defibrillation module 500, the parameter interface 600, the holding structure 141, and the treatment interface 700 can refer to the above mentioned door body 400, first insertion part 351, second insertion part 352, connection part 353, first guidance component 34, second guidance component 35, defibrillation module 500, parameter interface 600, holding structure 141, and treatment interface 700, respectively. The embodiments of this disclosure give no unnecessary details herein.

As shown in FIG. 17, the embodiment of this disclosure further provides a defibrillation device, including a host housing 100, a display component 200, and a recording instrument 300.

The host housing 100 serves as a housing of the entire defibrillation device, and includes a first outer wall 11a at a first side and a second outer wall 12a at a second side. That is to say, assuming that the first outer wall 11a is located at a front surface or front side of the host housing 100, the second outer wall 12a can be located at a bottom surface, a left surface, a right surface, a rear surface, or a top surface of the host housing 100. The embodiment of this disclosure does not limit this, of course, depending on the shape and placement state of the host housing 100, the first outer wall 11a may also be located at a side of the host housing 100, which side is inclined upwardly or downwardly, etc. The embodiments of this disclosure make no limitation for this. In the following, the embodiments of this disclosure continue to explain the first part 11 located at the front side of the host housing 100 as an example.

The display component 200 is assembled at the first outer wall 11a, which can be understood as being assembled at the front surface of the host housing 100.

The recording instrument 300 is at least partially assembled at the host housing 100, and configured to accommodate and print printing paper.

Wherein, the defibrillation device is configured such that the printing paper which is printed is capable of being outputted from the first side, which means that the defibrillation device is configured such that the printed paper is capable of being outputted from the front surface for the user to obtain the printed paper from the front surface. In addition, the defibrillation device is configured such that: the printing paper which is to be printed is capable of being placed into and taken out from the recording instrument 300 via the second side, such that there is no need to reserve a large area at the front surface of the host housing 100 for paper replacement. Based on this, the defibrillation device can also be configured with a display component 200 covering at least 50% of an outer surface of the first outer wall 11a, so that the display component 200 has a larger screen-to-body ratio at the front surface of the defibrillation device.

For example, the display component 200 covers 50%, 60%, and 71.4% of the outer surface of the first outer wall 11a, which is not limited by embodiments of this disclosure.

In some embodiments, the structures of the host housing 100, the display component 200, and the recording instrument 300 can respectively refer to the host housing 100, the display component 200, and the recording instrument 300 mentioned above. The first outer wall 11a can be the outer wall of the first part 11 facing the front surface, and the second outer wall 12a can be the outer wall of the second part 12 facing the bottom surface. The embodiments of this disclosure give no unnecessary details herein.

In some embodiments, the defibrillation device may further include one or more of the door body 400, the first insertion part 351, the second insertion part 352, the connection part 353, the first guide component 34, the second guide component 35, the defibrillation module 500, the parameter interface 600, the holding structure 141, and the treatment interface 700. The specific structures of the door body 400, the first insertion part 351, the second insertion part 352, the connection part 353, the first guide component 34, the second guide component 35, the defibrillation module 500, the parameter interface 600, the holding structure 141, and the treatment interface 700 can refer to the above mentioned door body 400, first insertion part 351, second insertion part 352, connection part 353, first guidance component 34, second guidance component 35, defibrillation module 500, parameter interface 600, holding structure 141, and treatment interface 700, respectively. The embodiments of this disclosure give no unnecessary details herein.

As shown in FIGS. 1 to 3, the embodiment of this disclosure also provides a defibrillation device, including a host housing 100, a display component 200, a recording instrument 300, and a door body 400.

The host housing 100 serves as a housing of the entire defibrillation device, and includes a first part 11 at a first side and a second part 12 at a second side which is adjacent to the first side. That is to say, assuming that the first part 11 is located at a front surface, or in other words at a front side of the host housing 100, the second part can be located at a bottom surface, a left surface, a right surface, or a top surface of the host housing 100. The embodiment of this disclosure does not limit this. But of course, depending on the shape and placement state of the host housing 100, The first part 11 may also be located at a side of the host housing 100, which is inclined upwardly or downwardly. The embodiments of this disclosure make no limitation for this. In the following, the embodiments of this disclosure continue to use the first part 11 located at the front side of the host housing 100 as an example for explanation.

The display component 200 can be assembled at the first part 11, which can be understood as being assembled at the front surface of the host housing 100.

The recording instrument 300 includes a housing 31 and a printing component 32. The housing 31 of the recording instrument is configured to accommodate printing paper, and the housing 31 of the recording instrument is at least partially assembled at the host housing 100. The recording instrument 300 is configured such that the printing paper inside the housing 31 of the recording instrument is capable of being outputted to the first side of the host housing 100. In other words, the printing paper inside the housing 31 of the recording instrument can be outputted from the front surface of the host housing 100, so as to facilitate user to obtain the printing paper. The printing component 32 is assembled at the housing 31 of the recording instrument and is located inside the host housing 100 for printing the printing paper accommodated inside the housing 31 of the recording instrument. The printing component 32 is arranged along a direction toward the second part 12, inclining toward or away from a plane in which the display component 20 is located. Alternatively, it can be understood that the printing component is inclined forwardly or backwardly from top to bottom. Compared to arranging the printing component 32 and the display component 200 side by side, a total height of the printing component 32 and the display component 200 in a vertical direction according to embodiments of this disclosure is lower, and thus the height of the first part **11** in the vertical direction can be made shorter. Therefore, assuming that the size of the display component 200 and the printing component 32 is constant, conversely speaking, the screen-to-body ratio of the display component 200 at the front side of the first part 11 can be larger.

The door body 400 is at least partially located at one side of the second part 12 of the host housing 100. The door body 400 is movably connected with the housing 31 of the recording instrument and/or the host housing 100, so as to enable the door body 400 to open or close an opening at the housing 31 of the recording instrument, thereby enabling that the printing paper is capable of being placed into or taken out from the housing 31 of the recording instrument via one side of the second part 12 of the host housing 100. In other words, the door body 400 used by the defibrillation device for replacing printing paper is at least partially located in an area which is outside the front surface of the host housing 100, which further enables that the printing paper is capable of being placed into or taken out from the recording instrument 300 via the area outside the front surface of the host housing 100. Therefore, compared to reserving a larger area at the front surface of the host housing 100 for paper replacement, the display component 200 at the front surface of the host housing 100 in embodiments of this disclosure can be made larger to increase the screen-to-body ratio at the front surface of the host housing 100.

From this, it can be seen that on the basis of outputting printing paper from the front surface of the host housing 100 for facilitating the user to obtain the printing paper, embodiments of this disclosure can increase the screen-to-body ratio at the front surface of the defibrillation device by inclining the printing component 32 and arranging the recording instrument 300 to replace the printing paper from surfaces other than the front surface of the host housing 100.

In some embodiments, the structures of the host housing 100, the display component 200, and the recording instrument 300 can respectively refer to the host housing 100, the display component 200, and the recording instrument 300 mentioned above. The first outer wall 11a can be the outer wall of the first part 11 facing the front surface, and the second outer wall 12a can be the outer wall of the second part 12 facing the bottom surface. The embodiments of this disclosure give no unnecessary details herein.

In some embodiments, the defibrillation device may further include one or more of the door body 400, the first insertion part 351, the second insertion part 352, the connection part 353, the first guide component 34, the second guide component 35, the defibrillation module 500, the parameter interface 600, the holding structure 141, and the treatment interface 700. The specific structures of the door body 400, first insertion part 351, the second insertion part 352, the connection part 353, the first guide component 34, the second guide component 35, the defibrillation module 500, the parameter interface 600, the holding structure 141, and the treatment interface 700 can refer to in the above mentioned door body 400, first insertion part 351, second insertion part 352, connection part 353, first guidance component 34, second guidance component 35, defibrillation module 500, parameter interface 600, holding structure 141, and treatment interface 700, respectively. The embodiments of this disclosure give no unnecessary details herein.

As shown in FIG. 17, the embodiment of this disclosure also provides a medical device, which can be a monitor, defibrillation device, etc. The embodiment of this disclosure does not limit this. The medical device can include a host housing 100 and a recording instrument 300.

The host housing 100 includes a first part 11 at a first side, and a second part 12 at a second side. The first part 11 has a first area 114 for fixing a display component 200. That is to say, assuming that the first part 11 is located at a front surface, or in other words at a front side of the host housing 100, the second part can be located at a bottom surface, a left surface, a right surface, a rear surface, or a top surface of the host housing 100. The embodiment of this disclosure does not limit this. But of course, depending on the shape and placement state of the host housing 100, the first part 11 may also be located at a side of the host housing 100, which is inclined upwardly or downwardly. The embodiments of this disclosure make no limitation for this. In the following, the embodiments of this disclosure continue to explain and illustrate the first part 11 located at the front surface of the host housing 100, which means that the front surface of the host housing 100 has a first area 114 for assembling and fixing the display component 200.

The recording instrument 300 is at least partially assembled inside the host housing 100, and is configured to accommodate and print printing paper.

Wherein, the medical device is configured such that: the printing paper which is printed is capable of being outputted from the first side. In other words, the medical device is configured such that: the printing paper which is printed is capable of being outputted from the front surface for facilitating user to obtain.

In additional, the medical device is also configured such that the printing paper which is to be printed is capable of being placed into and taken out from the recording instrument 300 via the second side of the host housing 100, thereby enabling the recording instrument 300 to be configured such that the printing paper is capable of being replaced from an area outside the front surface of the host housing 100. Therefore, compared to reserving a larger area at the front surface of the host housing 100 for paper replacement, the first area 114 at the front surface of the host housing 100 in embodiments of this disclosure can be made larger to assemble a larger display component 200.

At the same time, an orthographic projection of the recording instrument 300 on the first side at least partially overlaps with an orthographic projection of the first area 114 on the first side. Alternatively, it can be understood that the recording instrument 300 is at least partially located at a rear side of the first area 114. Such that, compared to arranging the recording instrument 300 and the first area 114 side by side at the front surface of the host housing 100, the first area 114 at the front surface of the host housing 100 in embodiments of this disclosure can be made larger.

From this, it can be seen that on the basis of outputting the printing paper from the front surface of the host housing 100 to facilitate user to obtain, the embodiment of this disclosure can reserve more space at the front surface of the medical device to assemble a larger display component 200 by hiding at least a portion of the recording instrument 300 behind the first area 114 and arranging the recording instrument 300 to replace the printing paper from surfaces other than the front surface of the host housing 100, thereby increasing the screen-to-body ratio at the front surface of the defibrillation device.

In some embodiments, the structures of the host housing 100, the display component 200, and the recording instrument 300 can respectively refer to the aforementioned host housing 100, display component 200, and recording instrument 300. The embodiments of this disclosure give no unnecessary details .

In some embodiments, the defibrillation device may further include one or more of the door body 400, the first insertion part 351, the second insertion part 352, the connection part 353, the first guide component 34, the second guide component 35, the defibrillation module 500, the parameter interface 600, the holding structure 141, and the treatment interface 700. Wherein the specific structures of the door body 400, the first insertion part 351, the second insertion part 352, the connection part 353, the first guide component 34, the second guide component 35, the defibrillation module 500, the parameter interface 600, the holding structure 141, and the treatment interface 700 can refer to the above mentioned door body 400, first insertion part 351, second insertion part 352, connection part 353, first guidance component 34, second guidance component 35, defibrillation module 500, parameter interface 600, holding structure 141, and treatment interface 700, respectively. The embodiments of this disclosure give no unnecessary details herein.

The embodiment of this disclosure also provides a recording instrument 300, which is applied to a medical device. The medical device can be a defibrillation device, a monitor, a sample analyzer, etc. The embodiment of this disclosure does not limit this.

As shown in FIGS. 10 and 11, the recording instrument 300 includes a housing 31, a recording instrument door 400a, and a handle 41.

The housing 31 of the recording instrument is provided with an accommodating cavity and a second opening, wherein the accommodating cavity is configured to accommodate printing paper, the second opening is configured such that the printing paper is capable of being placed into or taken out from the accommodating cavity, and the housing 31 is further provided with a first snap-fit 315. The door body 400a of the recording instrument is rotatably connected with the housing 31 of the recording instrument, so as to enable the door body 400a of the recording instrument to rotate in a first direction H1 to open the second opening, and to enable the door body 400a of the recording instrument to rotate in a second direction H2 to close the second opening. The first direction H1 is opposite to the second direction H2. It can also be understood as that the door body 400 can rotate forward to open the door and backward to close the door. The handle 41 is provided with a second snap-fit 411, and a fixed end of the handle 41 is rotatably connected with the door body 400a of the recording instrument. Wherein, the handle 41 is capable of rotating in a fourth direction, so as to enable a free end of the handle 41 to be adjacent to the door body 400a of recording instrument, and so as to enable the second snap-fit 411 and the first snap-fit 315 to be snap-fitted for locking the door body 400a of the recording instrument and the housing 31 of the recording instrument. The handle 41 is further capable of rotating in a third direction, so as to enable the free end of the handle 41 to be away from the door body 400a of recording instrument, and so as to enable the second snap-fit 411 and the first snap-fit 315 to be separated for unlocking the door body 400a of the recording instrument and the housing 31 of the recording instrument. The third direction H3 is opposite to the fourth direction H4.

Therefore, it can also be understood that when the handle 41 is rotated to be attached to the door body 400a of the recording instrument, the door body 400a of the recording instrument is locked accordingly, and when the handle 41 is rotated to stand up from the door body 400a of the recording instrument, the door body 400a of the recording instrument is unlocked accordingly. In this way, by setting a simple structure of the second snap-fit 411 at the handle 41, user can more conveniently unlock or lock the door body 400.

Wherein, the third direction H3 can be the same as the first direction H1, and the fourth direction H4 can be the same as the second direction H2.

Then, taking an example of the door body 400a of the recording instrument located at the bottom surface of the medical device, the door body 400a of the recording instrument is roughly horizontal when it is closed, and a fixed end of the door body 400a of the recording instrument is located at its rear side and rotatably connected with the housing 31 of the recording instrument. At this point, if the door body 400a of the recording instrument is rotated in the first direction H1 to open the door, the free end of the door body 400, or in other words, the front end of the door body 400, flips backwards. The handle 41 can be rotated in the third direction H3 until the free end of the handle 41 is far away from the door body 400, can be that the free end of the handle 41 flips backwards to stand up from the door body 400. Therefore, the user can flip backward the handle 41 to grasp it and then continue to flip the handle 41 backward to open the door body 400. Therefore, compared to pulling the handle 41 forward, left, or right before opening the door, and then pulling the handle backward to open the door, the door body 400 of embodiments of this disclosure is more convenient to open.

Alternatively, the third direction H3 can also be opposite to the first direction H1, and the fourth direction H4 is opposite to the second direction H2. The embodiment of this disclosure does not limit this.

In some embodiments, as shown in FIG. 12, the handle 41 is further provided with an abutting part. The abutting part is located at a side of the fixed end of the handle 41, which side back-faces the free end, and a distance from the fixed end of the handle 41 to the abutting part is smaller than a distance from the fixed end of the handle 41 to the free end of the handle 41. Furthermore, the handle 41 can be regarded as a lever as a whole, with the fixed end of the handle 41 being a fulcrum of the handle 41, and a first force arm formed from the abutting part to the fulcrum is smaller than a second arm formed from the free end of the handle 41 to the fulcrum. When the first snap-fit 315 and the second snap-fit 411 switch between snap-fitted and separation states, the abutting part 412 abuts against the first snap-fit 315.

That is to say, during the process of rotating the handle 41 to stand up relative to the door body 400, the first force arm can form a resistance arm, and the second force arm can form a power arm. Since the power arm is larger than the resistance arm, user can open the door through the handle 41 more effortlessly.

In some embodiments, the housing 31 of the recording instrument and the door body 400a of the recording instrument can refer to the aforementioned housing 31 of the recording instrument and door body 400, respectively. The embodiments of this disclosure give no unnecessary details.

In some embodiments, the recording instrument 300 may also include one or more of the printing component 32, the first insertion part 351, the second insertion part 352, the connection part 353, the first guide component 34, and the second guide component 35. The specific structures of the printing component 32, the first insertion part 351, the second insertion part 352, the connection part 353, the first guide component 34, and the second guide component 35 can refer to the above-mentioned printing component 32, first insertion part 351, second guide component 35, second insertion part 352, connection part 353, first guide component 34, and second guide component 35, respectively. The embodiments of this disclosure give no unnecessary details .

In the above embodiments, the descriptions of each embodiment have their own emphasis. For the parts that are not detailed in one embodiment, please refer to the relevant descriptions of other embodiments.

The above provides a detailed description for the defibrillation device, medical device, and recording instrument 300 provided in the embodiments of this disclosure. Specific examples are applied in this disclosure to explain its principles and implementation methods. The explanations of the above embodiments are only used to help understand the methods and core ideas of this disclosure. Meanwhile, for technical personnel in this field, there may be changes in the specific embodiments and application scope based on the ideas of this disclosure. In summary, the content of this description should not be understood as a limitation on this disclosure.

## Claims

1. A defibrillation device, comprising:
a host housing(100), which comprises a first part(11) at a first side and a second part(12) at a second side;
a display component(200), which is assembled at the first part(11);
a recording instrument(300), which comprises a housing(31) and a printing component(32), wherein the housing of the recording instrument(31) is configured to accommodate printing paper, and the housing of the recording instrument(31) is at least partially assembled at the host housing(100); wherein the recording instrument(300) is configured such that the printing paper inside the housing of the recording instrument(31) is capable of being outputted to the first side; wherein the printing component(32) is assembled at the housing of the recording instrument(31) and located at the host housing(100) to print the printing paper, which is accommodated inside the housing of the recording instrument(31); and
a door body(400), which is at least partially located at the second side and is movably connected with the housing of the recording instrument(31) and/or the host housing(100); wherein the door body(400) is capable of opening or closing an opening at the housing of the recording instrument(31), so as to enable the printing paper to be placed into and taken out from the housing of the recording instrument(31) via the second side;
**characterized in that**, an orthographic projection of the recording instrument(31) on the first side at least partially overlaps with an orthographic projection of the display component(200) on the first side.

2. The defibrillation device according to claim 1, **characterized in that**, an orthogonal projection of the housing of the recording instrument(31) on the first side at least partially overlaps with the orthographic projection of the display component(200) on the first side; and/or
an orthogonal projection of the printing component(32) on the first side at least partially overlaps with the orthographic projection of the display component(200) on the first side.

3. The defibrillation device according to claim 1, **characterized in that**, the second part(12) comprises an inward convex part(121), which extends from an outer surface of the second part(12) toward an interior of the host housing(100); wherein at least a portion of the inward convex part(121) is provided with a first convex structure(122) or a first concave structure, and the housing of the recording instrument(31) is provided with a second concave structure(311) or a second convex structure that fits with the first convex structure(122) or the first concave structure, so as to seal between the housing of the recording instrument(31) and the second part(12).

4. The defibrillation device according to claim 3, **characterized in that**, the inward convex part(121) comprises a first inside surface(1211) which faces the first part(11), the first convex structure(122) comprises a first convex rib(1221) which protrudes from the first inside surface(1211), the second concave structure(311) comprises a first groove(3111) with an opening which faces the first inside surface(1211), wherein the first convex rib(1221) is snapped into the first groove(3111).

5. The defibrillation device according to claim 3, **characterized in that**, the housing of the recording instrument(31) is provided with a first through-hole(312) that connects with an interior of the housing of the recording instrument(31) and the interior of the host housing(100), wherein a first fastener(33) penetrates through and is arranged inside the first through-hole(312), and the inward convex part(121) is provided with a first fastening structure(123), wherein the first fastener(33) is detachably connected with the first fastening structure(123).

6. The defibrillation device according to claim 4, **characterized in that**, the inward convex part(121) further comprises two second inside surfaces(1212), the housing of the recording instrument(31) is located between the two second inside surfaces(1212), which extend from different ends of the first inside surface(1211) toward the first part(11);
the first convex structure(122) further comprises two second convex ribs(1222), wherein each second inside surface(1212) is provided with one second convex rib(1222); the second concave structure(311) further comprises two second grooves(3112), wherein one second convex rib(1222) is snapped into one second groove(3112).

7. The defibrillation device according to claim 6, **characterized in that**, two pairs of position-limiting ribs(313,314) protrude from an outer surface of the housing of the recording instrument(31), each pair of position-limiting ribs(313,314) comprise a first position-limiting rib(313) and a second position-limiting rib(314), both pairs of position-limiting ribs(313,314) extend from the first inside surface(1211) toward the first part(11);
wherein the first position-limiting rib(313) in one pair of position-limiting ribs(313,314) abuts against a side of one second convex rib(1222), which side is adjacent to an outside of the second part(12); the second position-limiting rib(314) in said one pair of position-limiting ribs(313,314) is arranged at a side of said one second convex rib(1222), which side back-faces the first position-limiting rib(313); such that the second position-limiting rib(314) in said one pair of position-limiting ribs(313,314), said one second convex rib(1222) and the outer surface of the housing of the recording instrument(31), together form a first insertion groove;
wherein the first position-limiting rib(313) in the other pair of position-limiting ribs(313,314) abuts against a side of the other second convex rib(1222), which side is adjacent to the outside of the second part(12); the second position-limiting rib(314) in the other pair of position-limiting ribs(313,314) is arranged at a side of the other second convex rib(1222), which side back-faces the first position-limiting rib(313); such that the second position-limiting rib(314) in the other pair of position-limiting ribs(313,314), the other second convex rib(1222) and the outer surface of the housing of the recording instrument(31), together form a second insertion groove;
the defibrillation device further comprises a first insertion part(351) and a second insertion part(352), which are detachably inserted into the first insertion groove and the second insertion groove, respectively, so as to clamp the second convex ribs(1222) between the insertion parts(351,352) and the first position-limiting ribs(313);
preferably, the defibrillation device further comprises a connection part(353), which is partially located between the housing of the recording instrument(31) and the first part(11), and the connection part(353) is located between the two second inside surfaces(1212); wherein both ends of the connection part(353), which are respectively adjacent to each second inside surface(1212), are respectively connected with one of the insertion parts(351,352), so as to enable both ends of the connection part(353) to clamp the housing of the recording instrument(31) through the insertion parts(351,352); wherein a side of the connection part(353), which side back-faces the first inside surface(1211), is provided with a third convex structure or a third concave structure(3531), an inner surface of the first part(11) is provided with a fourth concave structure or a fourth convex structure(111) which fits with the third convex structure or the third concave structure(3531).

8. The defibrillation device according to claim 1, **characterized in that**, at least one of the housing of the recording instrument(31) and the host housing(100) is rotatably connected with the door body(400), so as to enable the door body(400) to rotate in a first direction to open the housing of the recording instrument(31), and so as to enable the door body(400) to rotate in a second direction to close the housing of the recording instrument(31); wherein the first direction is opposite to the second direction.

9. The defibrillation device according to claim 8, **characterized in that**, the door body(400) is provided with a handle(41), wherein a fixed end of the handle(41) is rotatably connected with the door body(400), so as to enable the handle(41) to rotate in a third direction until a free end of the handle(41) is away from the door body(400), and so as to enable the handle(41) to rotate in a fourth direction until the free end of the handle(41) is adjacent to the door body(400); wherein the third direction is same as the first direction, and the fourth direction is same as the second direction.

10. The defibrillation device according to claim 9, **characterized in that**, an inner wall of the housing of the recording instrument(31) is provided with a first snap-fit(315), and the handle(41) is provided with a second snap-fit(411); wherein the handle(41) is capable of rotating in the fourth direction until the first snap-fit(315) is snap-fitted with the second snap-fit(411), so as to lock the door body(400) and the housing of the recording instrument(31), and the handle(41) is further capable of rotating in the third direction to separate the first snap-fit(315) and the second snap-fit(411), so as to unlock the door body(400) and the housing of the recording instrument(31).

11. The defibrillation device according to claim 10, **characterized in that**,
the handle(41) is further provided with an abutting part(412); wherein the abutting part(412) is located at a side of the fixed end of the handle(41), which side back-faces the free end of the handle(41), wherein a distance from the fixed end of the handle(41) to the abutting part(412) is smaller than a distance from the fixed end of the handle(41) to the free end of the handle(41); wherein at least at one moment during the handle(41) rotating in the third direction, the abutting part(412) abuts against the housing of the recording instrument(31); or
an inner surface of the door body(400) is provided with a first guide component(34), which is configured to guide the printing paper inside the housing of the recording instrument(31) to an outside of the host housing(100); a side of the first guide component(34), which side faces the door body(400), is provided with a third groove(341), wherein the third groove(341) and the inner surface of the door body(400) together form a rotating hole; wherein the fixed end of the handle(41) is provided with a rotating shaft(413), which is rotatably assembled inside the rotating hole;
preferably the door body(400) is further provided with a second through-hole(42), wherein a second fastener(43) penetrates through and is arranged inside the second through-hole(42); the second fastener(43) is detachably connected with the first guide component(34), the handle(41) is capable of rotating in the fourth direction to cover the second through-hole; more preferably the door body(400) is further provided with a clamping part(44), and the rotating shaft(413) is rotatably clamped by the clamping part(44).

12. The defibrillation device according to claim 8, **characterized in that**, an inner surface of the door body(400) is provided with a first guide component(34), the housing of the recording instrument(31) is provided with a second guide component(35) that fits with the first guide component(34); wherein, when the door body(400) is rotated to close the housing of the recording instrument(31), the first guide component(34) and the second guide component(35) together form a paper output channel, wherein the paper output channel is configured to guide the printing paper inside the housing of the recording instrument(31) to be outputted to an outside of the host housing(100).

13. The defibrillation device according to claim 12, **characterized in that**, the first guide component(34) comprises at least one smooth surface, which is configured to form an inner surface of the paper output channel to support the printing paper; or
the recording instrument(300) further comprises a paper roller(36), which is rotatably assembled at the first guide component(34) to support the printing paper which passes through the first guide component(34), wherein the printing component(32) is configured to press and print the printing paper which is supported by the paper roller(36); or
the recording instrument(300) further comprises a paper roller(36), which is rotatably assembled at the housing of the recording instrument(31), the printing component(32) is assembled at the first guide component(34); wherein the paper roller(36) is configured to press the printing paper, which passes through the first guide component(34), onto the printing component(32) for printing.

14. The defibrillation device according to claim 12, **characterized in that**, a first paper output surface(354) is arranged at one end of the second guide component(35), which end is adjacent to the first part(11), and the door body(400) comprises a second paper output surface(45) that fits with the first paper output surface(354); wherein, when the door body(400) is rotated to close the housing of the recording instrument(31), the first paper output surface(354) and the second paper output surface(45) together form a paper output opening(452), wherein the paper output opening(452) is configured to guide the printing paper inside the paper output channel to be outputted to the outside of the host housing(100); wherein at least one of the first paper output surface(354) and the second paper output surface(45) is provided with a paper tearing structure(451);
preferably, at least one end of the second paper output surface(45) is provided with a concave-convex structure along a width direction of the printing paper to form the paper tearing structure, wherein a middle portion of the second paper output surface(45) along the width direction of the printing paper is a plane; or the first part(11) further comprises a sound output opening, which is arranged side by side with the paper output opening(452) at a same side of the display component(200).

15. The defibrillation device according to any one of claims 1-14, **characterized in that**, the host housing(100) further comprises a third part(13), a fourth part(14) and a fifth part(15); wherein the first part(11), the second part(12), the third part(13), the fourth part(14) and the fifth part(15) face different sides, respectively;
wherein the defibrillation device further comprises:
a defibrillation module(500), which is arranged inside the host housing(100), and configured to process state information of a human body and provide a defibrillation current and/or voltage;
a parameter interface(600), which is arranged at the third part(13) and connected with a detection component, wherein the parameter interface(600) is electrically connected with the defibrillation module(500), so as to enable the defibrillation module(500) to acquire the state information of the human body through the detection component;
a holding structure, which is arranged at the fourth part(14); and
a treatment interface(700), which is arranged at the fifth part(15) and connected with a defibrillation electrode, wherein the treatment interface(700) is electrically connected with the defibrillation module(500), so as to enable the defibrillation module(500) to apply the defibrillation current and/or voltage to the human body through the defibrillation electrode.

## Patentansprüche

1. Ein Defibrillationsvorrichtung, die Folgendes umfasst:
ein Hauptgehäuse (100), das einen ersten Teil (11) an einer ersten Seite und einen zweiten Teil (12) an einer zweiten Seite umfasst;
eine Anzeigekomponente (200), die an dem ersten Teil (11) angebracht ist;
ein Aufnahmeinstrument (300), das ein Gehäuse (31) und eine Druckkomponente (32) umfasst, wobei das Gehäuse des Aufnahmeinstruments (31) zur Aufnahme von Druckpapier ausgelegt ist und das Gehäuse des Aufnahmeinstruments (31) zumindest teilweise am Hauptgehäuse (100) montiert ist; wobei das Aufnahmeinstrument (300) so ausgelegt ist, dass das im Gehäuse des Aufnahmeinstruments (31) befindliche Druckpapier zur ersten Seite hin ausgegeben werden kann; wobei die Druckkomponente (32) am Gehäuse des Aufnahmeinstruments (31) montiert und am Hauptgehäuse (100) angeordnet ist, um das im Gehäuse des Aufnahmeinstruments (31) aufgenommene Druckpapier zu bedrucken; und
einen Türkörper (400), der sich zumindest teilweise auf der zweiten Seite befindet und beweglich mit dem Gehäuse des Aufnahmeinstruments (31) und/oder dem Hauptgehäuse (100) verbunden ist; wobei der Türkörper (400) in der Lage ist, eine Öffnung am Gehäuse des Aufnahmeinstruments (31) zu öffnen oder zu schließen, um das Einlegen und Entnehmen des Druckpapiers in das bzw. aus dem Gehäuse des Aufnahmeinstruments (31) über die zweite Seite zu ermöglichen;
**dadurch gekennzeichnet, dass** sich ein orthogonaler Vorsprung des Aufnahmeinstruments (31) auf der ersten Seite zumindest teilweise mit einem orthogonalen Vorsprung der Anzeigekomponente (200) auf der ersten Seite überlappt.

2. Die Defibrillationsvorrichtung nach Anspruch 1, **dadurch gekennzeichnet, dass** sich ein orthogonaler Vorsprung des Gehäuses des Aufnahmeinstruments (31) auf der ersten Seite sich zumindest teilweise mit dem orthogonalen Vorsprung der Anzeigekomponente (200) auf der ersten Seite überschneidet; und/oder
sich ein orthogonaler Vorsprung der Druckkomponente (32) auf der ersten Seite zumindest teilweise mit dem orthogonalen Vorsprung der Anzeigekomponente (200) auf der ersten Seite überlappt.

3. Die Defibrillationsvorrichtung nach Anspruch 1, **dadurch gekennzeichnet, dass** der zweite Teil (12) einen nach innen gewölbten Abschnitt (121) umfasst, der sich von einer Außenfläche des zweiten Teils (12) in Richtung des Inneren des Aufnahmegehäuses (100) erstreckt; wobei zumindest ein Abschnitt des nach innen gewölbten Abschnitts (121) mit einer ersten konvexen Struktur (122) oder einer ersten konkaven Struktur versehen ist, und das Gehäuse des Aufnahmeinstruments (31) mit einer zweiten konkaven Struktur (311) oder einer zweiten konvexen Struktur versehen ist, die mit der ersten konvexen Struktur (122) oder der ersten konkaven Struktur zusammenpasst, um eine Abdichtung zwischen dem Gehäuse des Aufnahmeinstruments (31) und dem zweiten Teil (12) herzustellen.

4. Die Defibrillationsvorrichtung nach Anspruch 3, **dadurch gekennzeichnet, dass** der nach innen gewölbte Abschnitt (121) eine erste Innenfläche (1211) aufweist, die dem ersten Abschnitt (11) zugewandt ist, die erste konvexe Struktur (122) eine erste konvexe Rippe (1221) aufweist, die von der ersten Innenfläche (1211) hervorsteht, die zweite konkave Struktur (311) eine erste Nut (311) mit einer Öffnung aufweist, die der ersten Innenfläche (1211) zugewandt ist, wobei die erste konvexe Rippe (1221) in die erste Nut (3111) eingerastet ist.

5. Die Defibrillationsvorrichtung nach Anspruch 3, **dadurch gekennzeichnet, dass** das Gehäuse des Aufnahmeinstruments (31) mit einer ersten Durchgangsbohrung (312) versehen ist, die den Innenraum des Gehäuses des Aufnahmeinstruments (31) mit dem Innenraum des Hauptgehäuses (100) verbindet, wobei ein erstes Befestigungselement (33) die erste Durchgangsbohrung (312) durchdringt und in deren Inneren angeordnet ist, und der nach innen gewölbte Abschnitt (121) mit einer ersten Befestigungsstruktur (123) versehen ist, wobei das erste Befestigungselement (33) lösbar mit der ersten Befestigungsstruktur (123) verbunden ist.

6. Die Defibrillationsvorrichtung nach Anspruch 4, **dadurch gekennzeichnet, dass** der nach innen gewölbte Abschnitt (121) ferner zwei zweite Innenflächen (1212) umfasst, das Gehäuse des Aufnahmeinstruments (31) zwischen den beiden zweiten Innenflächen (1212) angeordnet ist, die sich von unterschiedlichen Enden der ersten Innenfläche (1211) in Richtung des ersten Abschnitts (11) erstrecken;
die erste konvexe Struktur (122) ferner zwei zweite konvexe Rippen (1222) umfasst, wobei jede zweite Innenfläche (1212) mit einer zweiten konvexen Rippe (1222) versehen ist; die zweite konkave Struktur (311) ferner zwei zweite Nuten (3112) umfasst, wobei eine zweite konvexe Rippe (1222) in eine zweite Nut (3112) eingerastet ist.

7. Die Defibrillationsvorrichtung nach Anspruch 6, **dadurch gekennzeichnet, dass** zwei Paare von Positionsbegrenzungsrippen (313, 314) von einer Außenfläche des Gehäuses des Aufnahmeinstruments (31) vorstehen, jedes Paar von Positionsbegrenzungsrippen (313, 314) eine erste Positionsbegrenzungsrippe (313) und eine zweite Positionsbegrenzungsrippe (314) umfasst, sich beide Paare von Positionsbegrenzungsrippen (313, 314) von der ersten Innenfläche (1211) in Richtung des ersten Teils (11) erstrecken;
wobei die erste Positionsbegrenzungsrippe (313) in einem Paar von Positionsbegrenzungsrippen (313, 314) an einer Seite einer zweiten konvexen Rippe (1222) anliegt, wobei diese Seite an eine Außenseite des zweiten Teils (12) angrenzt; die zweite Positionsbegrenzungsrippe (314) in dem einen Paar von Positionsbegrenzungsrippen (313, 314) an einer Seite der einen zweiten konvexen Rippe (1222) angeordnet ist, wobei diese Seite der ersten Positionsbegrenzungsrippe (313) abgewandt ist; sodass die zweite Positionsbegrenzungsrippe (314) in dem genannten Paar von Positionsbegrenzungsrippen (313, 314), die genannte zweite konvexe Rippe (1222) und die Außenfläche des Gehäuses des Aufnahmeinstruments (31) gemeinsam eine erste Einführnut bilden;
wobei die erste Positionsbegrenzungsrippe (313) des anderen Paares von Positionsbegrenzungsrippen (313, 314) an einer Seite der anderen zweiten konvexen Rippe (1222) anliegt, wobei diese Seite an die Außenseite des zweiten Teils (12) angrenzt; die zweite Positionsbegrenzungsrippe (314) des anderen Paares von Positionsbegrenzungsrippen (313, 314) an einer Seite der anderen zweiten konvexen Rippe (1222) angeordnet ist, wobei diese Seite der ersten Positionsbegrenzungsrippe (313) abgewandt ist; sodass die zweite Positionsbegrenzungsrippe (314) des anderen Paares von Positionsbegrenzungsrippen (313, 314), die andere zweite konvexe Rippe (1222) und die Außenfläche des Gehäuses des Aufnahmeinstruments (31) gemeinsam eine zweite Einführnut bilden;
die Defibrillationsvorrichtung ferner ein erstes Einführteil (351) umfasst und ein zweites Einführteil (352), die lösbar in die erste Einführnut bzw. die zweite Einführnut eingeführt werden, um die zweiten konvexen Rippen (1222) zwischen den Einführteilen (351, 352) und den ersten Positionsbegrenzungsrippen (313) festzuklemmen;
die Defibrillationsvorrichtung vorzugsweise ferner ein Verbindungsteil (353) umfasst, das sich teilweise zwischen dem Gehäuse des Aufnahmeinstruments (31) und dem ersten Teil (11) befindet, wobei sich das Verbindungsteil (353) zwischen den beiden zweiten Innenflächen (1212) befindet; wobei beide Enden des Verbindungsteils (353), die jeweils an eine der zweiten Innenflächen (1212) angrenzen, jeweils mit einem der Einsteckteile (351, 352) verbunden sind, sodass beide Enden des Verbindungsteils (353) das Gehäuse des Aufnahmeinstruments (31) über die Einsteckteile (351, 352) festklemmen können; wobei eine Seite des Verbindungsteils (353), die der ersten Innenfläche (1211) abgewandt ist, mit einer dritten konvexen Struktur oder einer dritten konkaven Struktur (3531) versehen ist, und eine Innenfläche des ersten Teils (11) mit einer vierten konkaven Struktur oder einer vierten konvexen Struktur (111) versehen ist, die mit der dritten konvexen Struktur oder der dritten konkaven Struktur(3531) zusammenpasst.

8. Die Defibrillationsvorrichtung nach Anspruch 1, **dadurch gekennzeichnet, dass** zumindest eines von dem Gehäuse des Aufnahmeinstruments (31) und dem Hauptgehäuse (100) drehbar mit dem Türkörper (400) verbunden ist, um eine Drehung des Türkörpers (400) in eine erste Richtung zum Öffnen des Gehäuses des Aufnahmeinstruments (31) sowie eine Drehung des Türkörpers (400) in eine zweite Richtung zum Schließen des Gehäuses des Aufnahmeinstruments (31) zu ermöglichen; wobei die erste Richtung der zweiten Richtung entgegengesetzt ist.

9. Die Defibrillationsvorrichtung nach Anspruch 8, **dadurch gekennzeichnet, dass** der Türkörper (400) mit einem Griff (41) versehen ist, wobei ein feststehendes Ende des Griffs (41) drehbar mit dem Türkörper (400) verbunden ist, sodass sich der Griff (41) in eine dritte Richtung drehen kann, bis sich ein freies Ende des Griffs (41) vom Türkörper (400) entfernt hat, und der Griff (41) sich in eine vierte Richtung drehen kann, bis das freie Ende des Griffs (41) an den Türkörper (400) angrenzt; wobei die dritte Richtung mit der ersten Richtung übereinstimmt und die vierte Richtung mit der zweiten Richtung übereinstimmt.

10. Die Defibrillationsvorrichtung nach Anspruch 9, **dadurch gekennzeichnet, dass** eine Innenwand des Gehäuses des Aufnahmeinstruments (31) mit einer ersten Rastverbindung (315) versehen ist und der Griff (41) mit einer zweiten Rastverbindung (411) versehen ist; wobei der Griff (41) in die vierte Richtung gedreht werden kann, bis die erste Rastvorrichtung (315) mit der zweiten Rastvorrichtung (411) eingerastet ist, um den Türkörper (400) und das Gehäuse des Aufnahmeinstruments (31) zu verriegeln, und der Griff (41) ferner in der dritten Richtung drehbar ist, um die erste Rastverbindung (315) und die zweite Rastverbindung (411) zu lösen, um den Türkörper (400) und das Gehäuse des Aufnahmeinstruments (31) zu entriegeln.

11. Die Defibrillationsvorrichtung nach Anspruch 10, **dadurch gekennzeichnet, dass** der Griff (41) ferner mit einem Anschlagteil (412) versehen ist; wobei sich das Anschlagteil (412) an einer Seite des festen Endes des Griffs (41) befindet, die dem freien Ende des Griffs (41) abgewandt ist, wobei ein Abstand vom festen Ende des Griffs (41) zum Anschlagteil (412) kleiner ist als ein Abstand vom festen Ende des Griffs (41) zum freien Ende des Griffs (41); wobei zumindest zu einem Zeitpunkt während der Drehung des Griffs (41) in die dritte Richtung der Anschlagabschnitt (412) am Gehäuse des Aufnahmeinstruments (31) anliegt; oder
eine Innenfläche des Türkörpers (400) mit einem ersten Führungselement (34) versehen ist, das so ausgebildet ist, dass es das Druckpapier innerhalb des Gehäuses des Aufnahmeinstruments (31) zu einer Außenseite des Hauptgehäuses (100) führt; eine Seite der ersten Führungskomponente (34), die dem Türkörper (400) zugewandt ist, mit einer dritten Nut (341) versehen ist, wobei die dritte Nut (341) und die Innenfläche des Türkörpers (400) gemeinsam eine Drehbohrung bilden; wobei das feststehende Ende des Griffs (41) mit einer Drehwelle (413) versehen ist, die drehbar in der Drehbohrung montiert ist;
vorzugsweise der Türkörper (400) ferner mit einer zweiten Durchgangsbohrung (42) versehen ist, wobei ein zweites Befestigungselement (43) die zweite Durchgangsbohrung (42) durchdringt und in dessen Innerem angeordnet ist; das zweite Befestigungselement (43) lösbar mit dem ersten Führungsbauteil (34) verbunden ist, wobei der Griff (41) in die vierte Richtung gedreht werden kann, um die zweite Durchgangsbohrung abzudecken; noch vorzugsweises der Türkörper (400) ferner mit einem Klemmteil (44) versehen ist und die Drehwelle (413) durch das Klemmteil (44) drehbar geklemmt wird.

12. Die Defibrillationsvorrichtung nach Anspruch 8, **dadurch gekennzeichnet, dass** eine Innenfläche des Türkörpers (400) mit einer ersten Führungskomponente (34) versehen ist, das Gehäuse des Aufnahmeinstruments (31) mit einer zweiten Führungskomponente (35) versehen ist, die mit der ersten Führungskomponente (34) zusammenpasst; wobei, wenn der Türkörper (400) gedreht wird, um das Gehäuse des Aufnahmeinstruments (31) zu schließen, das erste Führungselement (34) und das zweite Führungselement (35) gemeinsam einen Papierausgabekanal bilden, wobei der Papierausgabekanal so ausgebildet ist, dass er das Druckpapier im Inneren des Gehäuses des Aufnahmeinstruments (31) so führt, dass es aus dem Hauptgehäuse (100) nach außen ausgegeben wird.

13. Die Defibrillationsvorrichtung nach Anspruch 12, **dadurch gekennzeichnet, dass** die erste Führungskomponente (34) mindestens eine glatte Oberfläche aufweist, die so ausgebildet ist, dass sie eine Innenfläche des Papierausgabekanals bildet, um das Druckpapier abzustützen; oder
das Aufnahmeinstrument (300) ferner eine Papierwalze (36) umfasst, die drehbar an der ersten Führungskomponente (34) angebracht ist, um das durch die erste Führungskomponente (34) laufende Druckpapier zu stützen, wobei die Druckkomponente (32) so ausgelegt ist, dass sie das von der Papierwalze (36) gestützte Druckpapier andrückt und bedruckt; oder
das Aufnahmeinstrument (300) ferner eine Papierwalze (36) umfasst, die drehbar am Gehäuse des Aufnahmeinstruments (31) angebracht ist, wobei die Druckkomponente (32) an der ersten Führungskomponente (34) angebracht ist; wobei die Papierwalze (36) so ausgelegt ist, dass sie das Druckpapier, das durch die erste Führungskomponente (34) läuft, zum Bedrucken auf die Druckkomponente (32) drückt.

14. Die Defibrillationsvorrichtung nach Anspruch 12, **dadurch gekennzeichnet, dass** an einem Ende der zweiten Führungskomponente (35), das an den ersten Teil (11) angrenzt, eine erste Papierausgabefläche (354) angeordnet ist und der Türkörper (400) eine zweite Papierausgabefläche (45) umfasst, die mit der ersten Papierausgabefläche (354) zusammenpasst; wobei, wenn der Türkörper (400) gedreht wird, um das Gehäuse des Aufnahmeinstruments (31) zu schließen, die erste Papierausgabefläche (354) und die zweite Papierausgabefläche (45) gemeinsam eine Papierausgabeöffnung (452) bilden, wobei die Papierausgabeöffnung (452) so ausgebildet ist, dass sie das Druckpapier innerhalb des Papierausgabekanals so führt, dass es aus dem Hauptgehäuse (100) nach außen ausgegeben wird; wobei mindestens eine der ersten Papierausgabeflächen (354) und der zweiten Papierausgabeflächen (45) mit einer Papierabrissstruktur (451) versehen ist;
vorzugsweise mindestens ein Ende der zweiten Papierausgabefläche (45) mit einer konkav-konvexen Struktur entlang der Breitenrichtung des Druckpapiers versehen ist, um die Papierabrissstruktur zu bilden, wobei ein mittlerer Abschnitt der zweiten Papierausgabefläche (45) entlang der Breitenrichtung des Druckpapiers eine Ebene bildet; oder der erste Teil (11) ferner eine Tonausgabeöffnung umfasst, die nebeneinander mit der Papierausgabeöffnung (452) auf derselben Seite der Anzeigekomponente (200) angeordnet ist.

15. Die Defibrillationsvorrichtung nach einem der Ansprüche 1 bis 14, **dadurch gekennzeichnet, dass** das Hauptgehäuse (100) ferner einen dritten Teil (13), einen vierten Teil (14) und einen fünften Teil (15) umfasst; wobei der erste Teil (11), der zweite Teil (12), der dritte Teil (13), der vierte Teil (14) und der fünfte Teil (15) jeweils zu unterschiedlichen Seiten weisen;
wobei die Defibrillationsvorrichtung ferner Folgendes umfasst:
ein Defibrillationsmodul (500), das innerhalb des Hauptgehäuses (100) angeordnet und so ausgelegt ist, dass es Zustandsinformationen eines menschlichen Körpers verarbeitet und einen Defibrillationsstrom und/oder eine Defibrillationsspannung bereitstellt;
eine Parameterschnittstelle (600), die am dritten Teil (13) angeordnet und mit einer Erfassungskomponente verbunden ist, wobei die Parameterschnittstelle (600) elektrisch mit dem Defibrillationsmodul (500) verbunden ist, um es dem Defibrillationsmodul (500) zu ermöglichen, die Zustandsinformationen des menschlichen Körpers über die Erfassungskomponente zu erfassen;
eine Haltevorrichtung, die am vierten Teil (14) angeordnet ist; und
eine Behandlungsschnittstelle (700), die am fünften Teil (15) angeordnet und mit einer Defibrillationselektrode verbunden ist, wobei die Behandlungsschnittstelle (700) elektrisch mit dem Defibrillationsmodul (500) verbunden ist, um es dem Defibrillationsmodul (500) zu ermöglichen, den Defibrillationsstrom und/oder die Defibrillationsspannung über die Defibrillationselektrode an den menschlichen Körper anzulegen.

## Revendications

1. Dispositif de défibrillation, comprenant :
un logement hôte (100), lequel comprend une première partie (11) au niveau d'un premier côté et une deuxième partie (12) au niveau d'un deuxième côté ;
un composant d'affichage (200), lequel est assemblé au niveau de la première partie (11) ;
un instrument d'enregistrement (300), lequel comprend un logement (31) et un composant d'impression (32), où le logement de l'instrument d'enregistrement (31) est configuré pour recevoir du papier d'impression, et le logement de l'instrument d'enregistrement (31) est au moins en partie assemblé au niveau du logement hôte (100) ; où l'instrument d'enregistrement (300) est configuré de telle sorte que le papier d'impression à l'intérieur du logement de l'instrument d'enregistrement (31) peut être délivré sur le premier côté ; où le composant d'impression (32) est assemblé au niveau du logement de l'instrument d'enregistrement (31) et situé au niveau du logement hôte (100) pour imprimer sur le papier d'impression, lequel est reçu à l'intérieur du logement de l'instrument d'enregistrement (31) ; et
un corps de porte (400), lequel est au moins en partie situé au niveau du deuxième côté et est relié de manière mobile au logement de l'instrument d'enregistrement (31) et/ou au logement hôte (100) ; où le corps de porte (400) peut ouvrir ou fermer une ouverture au niveau du logement de l'instrument d'enregistrement (31), de sorte à permettre au papier d'impression d'être placé dans et d'être extrait du logement de l'instrument d'enregistrement (31) via le deuxième côté ;
**caractérisé en ce qu'**une projection orthographique de l'instrument d'enregistrement (31) sur le premier côté chevauche au moins en partie une projection orthographique du composant d'affichage (200) sur le premier côté.

2. Dispositif de défibrillation selon la revendication 1, **caractérisé en ce qu'**une projection orthogonale du logement de l'instrument d'enregistrement (31) sur le premier côté chevauche au moins en partie la projection orthographique du composant d'affichage (200) sur le premier côté ; et/ou
une projection orthogonale du composant d'impression (32) sur le premier côté chevauche au moins en partie la projection orthographique du composant d'affichage (200) sur le premier côté.

3. Dispositif de défibrillation selon la revendication 1, **caractérisé en ce que** la deuxième partie (12) comprend une partie convexe vers l'intérieur (121), laquelle s'étend depuis une surface extérieure de la deuxième partie (12) vers un intérieur du logement hôte (100) ; où au moins une portion de la partie convexe vers l'intérieur (121) est dotée d'une première structure convexe (122) ou d'une première structure concave, et le logement de l'instrument d'enregistrement (31) est doté d'une seconde structure concave (311) ou d'une seconde structure convexe qui coïncide avec la première structure convexe (122) ou la première structure concave, de sorte à former un joint entre le logement de l'instrument d'enregistrement (31) et la deuxième partie (12).

4. Dispositif de défibrillation selon la revendication 3, **caractérisé en ce que** la partie convexe vers l'intérieur (121) comprend une première surface intérieure (1211) laquelle fait face à la première partie (11), la première structure convexe (122) comprend une première nervure convexe (1221) laquelle dépasse de la première surface intérieure (1211), la seconde structure concave (311) comprend une première rainure (3111) avec une ouverture qui fait face à la première surface intérieure (1211), où la première nervure convexe (1221) est emboîtée dans la première rainure (3111).

5. Dispositif de défibrillation selon la revendication 3, **caractérisé en ce que** le logement de l'instrument d'enregistrement (31) est doté d'un premier trou traversant (312), qui est relié à un intérieur du logement de l'instrument d'enregistrement (31) et à l'intérieur du logement hôte (100), où une première fixation (33) pénètre à travers et est agencée à l'intérieur du premier trou traversant (312), et la partie convexe vers l'intérieur (121) est dotée d'une première structure de fixation (123), où la première fixation (33) est reliée de manière amovible à la première structure de fixation (123).

6. Dispositif de défibrillation selon la revendication 4, **caractérisé en ce que** la partie convexe vers l'intérieur (121) comprend en outre deux secondes surfaces intérieures (1212), le logement de l'instrument d'enregistrement (31) est situé entre les deux secondes surfaces intérieures (1212), lesquelles s'étendent depuis différentes extrémités de la première surface intérieure (1211) vers la première partie (11) ;
la première structure convexe (122) comprend en outre deux secondes nervures convexes (1222), où chaque seconde surface intérieure (1212) est dotée d'une seconde nervure convexe (1222) ; la seconde structure concave (311) comprend en outre deux secondes rainures (3112), où une seconde nervure convexe (1222) est emboîtée dans une seconde rainure (3112).

7. Dispositif de défibrillation selon la revendication 6, **caractérisé en ce que** deux paires de nervures de délimitation de position (313, 314) dépassent d'une surface extérieure du logement de l'instrument d'enregistrement (31), chaque paire de nervures de délimitation de position (313, 314) comprend une première nervure de délimitation de position (313) et une seconde nervure de délimitation de position (314), les paires de nervures de délimitation de position (313, 314) s'étendent toutes deux depuis la première surface intérieure (1211) vers la première partie (11) ;
où la première nervure de délimitation de position (313) dans une première paire de nervures de délimitation de position (313, 314) bute contre un côté d'une première seconde nervure convexe (1222), lequel côté est adjacent à un extérieur de la deuxième partie (12) ; la seconde nervure de délimitation de position (314) dans ladite première paire de nervures de délimitation de position (313, 314) est agencée au niveau d'un côté de ladite première seconde nervure convexe (1222), lequel côté est orienté à l'opposé de la première nervure de délimitation de position (313) ; de telle sorte que la seconde nervure de délimitation de position (314) dans ladite première paire de nervures de délimitation de position (313, 314), ladite première seconde nervure convexe (1222) et la surface extérieure du logement de l'instrument d'enregistrement (31) forment ensemble une première rainure d'insertion ;
où la première nervure de délimitation de position (313) dans l'autre paire de nervures de délimitation de position (313, 314) bute contre un côté de l'autre seconde nervure convexe (1222), lequel côté est adjacent à l'extérieur de la deuxième partie (12) ; la seconde nervure de délimitation de position (314) dans l'autre paire de nervures de délimitation de position (313, 314) est agencée au niveau d'un côté de l'autre seconde nervure de délimitation de position (1222), lequel côté est orienté à l'opposé de la première nervure de délimitation de position (313) ; de telle sorte que la seconde nervure de délimitation de position (314) dans l'autre paire de nervures de délimitation de position (313, 314), l'autre seconde nervure convexe (1222) et la surface extérieure du logement de l'instrument d'enregistrement (31) forment ensemble une seconde rainure d'insertion ;
le dispositif de défibrillation comprend en outre une première partie d'insertion (351) et une seconde partie d'insertion (352), lesquelles sont insérées de manière amovible dans la première rainure d'insertion et la seconde rainure d'insertion, respectivement, de sorte à serrer les secondes nervures convexes (1222) entre les parties d'insertion (351, 352) et les premières nervures de délimitation de position (313) ;
de préférence, le dispositif de défibrillation comprend en outre une partie de liaison (353), laquelle est en partie située entre le logement de l'instrument d'enregistrement (31) et la première partie (11), et la partie de liaison (353) est située entre les deux secondes surfaces intérieures (1212) ; où les deux extrémités de la partie de liaison (353), lesquelles sont respectivement adjacentes à chaque seconde surface intérieure (1212), sont respectivement reliées à l'une des parties d'insertion (351, 352), de sorte à permettre aux deux extrémités de la partie de liaison (353) de serrer le logement de l'instrument d'enregistrement (31) par le biais des parties d'insertion (351, 352) ; où un côté de la partie de liaison (353), lequel côté est orienté à l'opposé de la première surface intérieure (1211), est doté d'une troisième structure convexe ou d'une troisième structure concave (3531), une surface intérieure de la première partie (11) est dotée d'une quatrième structure concave ou d'une quatrième structure convexe (111) laquelle coïncide avec la troisième structure convexe ou la troisième structure concave (3531).

8. Dispositif de défibrillation selon la revendication 1, **caractérisé en ce qu'**au moins l'un du logement de l'instrument d'enregistrement (31) et du logement hôte (100) est relié avec possibilité de rotation au corps de porte (400), de sorte à permettre au corps de porte (400) de tourner dans un premier sens pour ouvrir le logement de l'instrument d'enregistrement (31), et de sorte à permettre au corps de porte (400) de tourner dans un second sens pour fermer le logement de l'instrument d'enregistrement (31) ; où le premier sens est opposé au second sens.

9. Dispositif de défibrillation selon la revendication 8, **caractérisé en ce que** le corps de porte (400) est doté d'une poignée (41), où une extrémité fixée de la poignée (41) est reliée avec possibilité de rotation au corps de porte (400), de sorte à permettre à la poignée (41) de tourner dans un troisième sens jusqu'à ce qu'une extrémité libre de la poignée (41) soit écartée du corps de porte (400), et de sorte à permettre à la poignée (41) de tourner dans un quatrième sens jusqu'à ce que l'extrémité libre de la poignée (41) soit adjacente au corps de porte (400) ; où le troisième sens est le même que le premier sens et le quatrième sens est le même que le second sens.

10. Dispositif de défibrillation selon la revendication 9, **caractérisé en ce qu'**une paroi intérieure du logement de l'instrument d'enregistrement (31) est dotée d'un premier élément d'emboîtement (315), et la poignée (41) est dotée d'un second élément d'emboîtement (411) ; où la poignée (41) peut tourner dans le quatrième sens jusqu'à ce que le premier élément d'emboîtement (315) soit emboîté avec le second élément d'emboîtement (411), de sorte à verrouiller le corps de porte (400) et le logement de l'instrument d'enregistrement (31), et la poignée (41) peut en outre tourner dans le troisième sens pour séparer le premier élément d'emboîtement (315) et le second élément d'emboîtement (411), de sorte à déverrouiller le corps de porte (400) et le logement de l'instrument d'enregistrement (31).

11. Dispositif de défibrillation selon la revendication 10, **caractérisé en ce que**
la poignée (41) est en outre dotée d'une partie de butée (412) ; où la partie de butée (412) est située au niveau d'un côté de l'extrémité fixée de la poignée (41), lequel côté est orienté à l'opposé de l'extrémité libre de la poignée (41), où une distance de l'extrémité fixée de la poignée (41) à la partie de butée (412) est inférieure à une distance de l'extrémité fixée de la poignée (41) à l'extrémité libre de la poignée (41) ; où au moins à un moment durant la rotation de la poignée (41) dans le troisième sens, la partie de butée (412) bute contre le logement de l'instrument d'enregistrement (31) ; ou
une surface intérieure du corps de porte (400) est dotée d'un premier composant de guidage (34), lequel est configuré pour guider le papier d'impression à l'intérieur du logement de l'instrument d'enregistrement (31) vers un extérieur du logement hôte (100) ; un côté du premier composant de guidage (34), lequel côté fait face au corps de porte (400) est doté d'une troisième rainure (341), laquelle troisième rainure (341) et la surface intérieure du corps de porte (400) forment ensemble un trou de rotation ; où l'extrémité fixée de la poignée (41) est dotée d'un axe de rotation (413), lequel est assemblé avec possibilité de rotation dans le trou de rotation ;
de préférence, le corps de porte (400) est en outre doté d'un second trou traversant (42), où une seconde fixation (43) pénètre à travers et est agencée à l'intérieur du second trou traversant (42) ; la seconde fixation (43) est reliée de manière amovible au premier composant de guidage (34), la poignée (41) peut tourner dans le quatrième sens pour couvrir le second trou traversant ; de façon davantage préférée, le corps de porte (400) est en outre doté d'une partie de serrage (44), et l'axe de rotation (413) est serré avec possibilité de rotation par la partie de serrage (44).

12. Dispositif de défibrillation selon la revendication 8, **caractérisé en ce qu'**une surface intérieure du corps de porte (400) est dotée d'un premier composant de guidage (34), le logement de l'instrument d'enregistrement (31) est doté d'un second composant de guidage (35) qui coïncide avec le premier composant de guidage (34) ; où, lorsque le corps de porte (400) est tourné pour fermer le logement de l'instrument d'enregistrement (31), le premier composant de guidage (34) et le second composant de guidage (35) forment ensemble un canal de sortie de papier, où le canal de sortie de papier est configuré pour guider le papier d'impression à l'intérieur du logement de l'instrument d'enregistrement (31) pour le délivrer à un extérieur du logement hôte (100).

13. Dispositif de défibrillation selon la revendication 12, **caractérisé en ce que** le premier composant de guidage (34) comprend au moins une surface lisse, laquelle est configurée pour former une surface intérieure du canal de sortie de papier pour supporter le papier d'impression ; ou
l'instrument d'enregistrement (300) comprend en outre un rouleau de papier (36), lequel est assemblé avec possibilité de rotation au niveau du premier composant de guidage (34) pour supporter le papier d'impression lequel passe par le premier composant de guidage (34), où le composant d'impression (32) est configuré pour appuyer et imprimer sur le papier d'impression lequel est supporté par le rouleau de papier (36) ; ou
l'instrument d'enregistrement (300) comprend en outre un rouleau de papier (36), lequel est assemblé avec possibilité de rotation au niveau du logement de l'instrument d'enregistrement (31), le composant d'impression (32) est assemblé au niveau du premier composant de guidage (34) ; où le rouleau de papier (36) est configuré pour appuyer le papier d'impression, lequel passe par le premier composant de guidage (34), sur le composant d'impression (32) pour impression.

14. Dispositif de défibrillation selon la revendication 12, **caractérisé en ce qu'**une première surface de sortie de papier (354) est agencée au niveau d'une première extrémité du second composant de guidage (35), laquelle extrémité est adjacente à la première partie (11), et le corps de porte (400) comprend une seconde surface de sortie de papier (45) qui coïncide avec la première surface de sortie de papier (354) ; où, lorsque le corps de porte (400) est tourné pour fermer le logement de l'instrument d'enregistrement (31), la première surface de sortie de papier (354) et la seconde surface de sortie de papier (45) forment ensemble une ouverture de sortie de papier (452), où l'ouverture de sortie de papier (452) est configurée pour guider le papier d'impression à l'intérieur du canal de sortie de papier pour le délivrer à l'extérieur du logement hôte (100) ; où au moins l'une de la première surface de sortie de papier (354) et de la seconde surface de sortie de papier (45) est dotée d'une structure de déchirement de papier (451) ;
de préférence, au moins une extrémité de la seconde surface de sortie de papier (45) est dotée d'une structure convexe-concave le long d'une direction de largeur du papier d'impression pour former la structure de déchirement de papier, où une portion centrale de la seconde surface de sortie de papier (45) le long de la direction de largeur du papier d'impression est un plan ; ou la première partie (11) comprend en outre une ouverture de sortie sonore, laquelle est agencée côte à côte de l'ouverture de sortie de papier (452) d'un même côté du composant d'affichage (200).

15. Dispositif de défibrillation selon l'une quelconque des revendications 1 à 14, **caractérisé en ce que** le logement hôte (100) comprend en outre une troisième partie (13), une quatrième partie (14) et une cinquième partie (15) ; où la première partie (11), la deuxième partie (12), la troisième partie (13), la quatrième partie (14) et la cinquième partie (15) font face à différents côtés, respectivement ;
où le dispositif de défibrillation comprend en outre :
un module de défibrillation (500), lequel est agencé à l'intérieur du logement hôte (100), et configuré pour traiter des informations d'état d'un corps humain et fournir un courant et/ou une tension de défibrillation ;
une interface de paramètre (600), laquelle est agencée au niveau de la troisième partie (13) et est reliée à un composant de détection, où l'interface de paramètre (600) est électriquement reliée au module de défibrillation (500), de sorte à permettre au module de défibrillation (500) d'acquérir les informations d'état du corps humain par le biais du composant de détection ;
une structure de tenue, laquelle est agencée au niveau de la quatrième partie (14) ; et
une interface de traitement (700), laquelle est agencée au niveau de la cinquième partie (15) et reliée à une électrode de défibrillation, où l'interface de traitement (700) est électriquement reliée au module de défibrillation (500), de sorte à permettre au module de défibrillation (500) d'appliquer le courant et/ou la tension de défibrillation au corps humain par le biais de l'électrode de défibrillation.
